# EUROPEAN PATENT SPECIFICATION

(11) **EP 1 470 127 B1**
(45) Date of publication and mention of the grant of the patent: **02.05.2007**
(21) Application number: 03700767.1
(22) Date of filing: 29.01.2003
(51) Int. Cl.: C07D 487/22, A61K 31/40

(54) **N, N'-DIMETHYLATED N-CONFUSED PORPHYRINS**
N,N-DIMETHYLIERTE N-INVERTIERTE PORPHYRINE
PORPHYRINES N,N'-DIMETHYLEES N-COCONDENSEES

(30) Priority: 29.01.2002 US 352956 P
(43) Date of publication of application: 27.10.2004
(73) Proprietor: THE UNIVERSITY OF BRITISH COLUMBIA, Vancouver, British Columbia V6T 1Z3 (CA)
(72) Inventor: DOLPHIN, David, H., Vancouver, British Columbia V6R 2R2 (CA); XIAO, Ziwei, Cambridge, MA 02138 (US)
(74) Representative: Fisher, Adrian John
(86) International application number: PCT/CA2003/000120
(87) International publication number: WO 2003/064426

(56) References cited:
- WO-A-01/53300
- CHMIELEWSKI, P.J. ET AL.: "Copper(II) Complexes of Inverted Porphyrin and Its Methylated Derivatives" INORGANIC CHEMISTRY, vol. 39, pages 5475-5482, XP002239634 cited in the application
- CHMIELEWSKI, P.J. ET AL.: "Reactions of Nickel(II) 2-Aza-5,10,15,20-tetraphenyl-21- carbaporphyrin with Methyl Iodide. The First Structural Characterization of a Paramagnetic Organometallic Nickel(II) Complex" JOURNAL OF THE AMERICAN CHEMICAL SOCIETY, vol. 118, pages 5690-5701, XP002239635 cited in the application
- FURUTA, H. ET AL.: ""N-Confused Porphyrin": A New Isomer of Tetraphenylporphyrin" JOURNAL OF THE AMERICAN CHEMICAL SOCIETY, vol. 116, pages 767-768, XP002239636 cited in the application

## Description

### Technical Field

The present invention provides a novel class of therapeutic macrocycle compounds useful in photodynamic therapy that are based on the N-confused porphyrin ring system. The macrocycle compounds contain both outer and inner ring methyl groups and have absorption spectra suitable for therapeutic use in tissues. The invention also provides a demonstration of their capability of generating singlet oxygen and thus their use in therapeutic contexts such as photodynamic therapy (PDT). Methods for the preparation of the novel class of compounds are also provided.

### Background Art

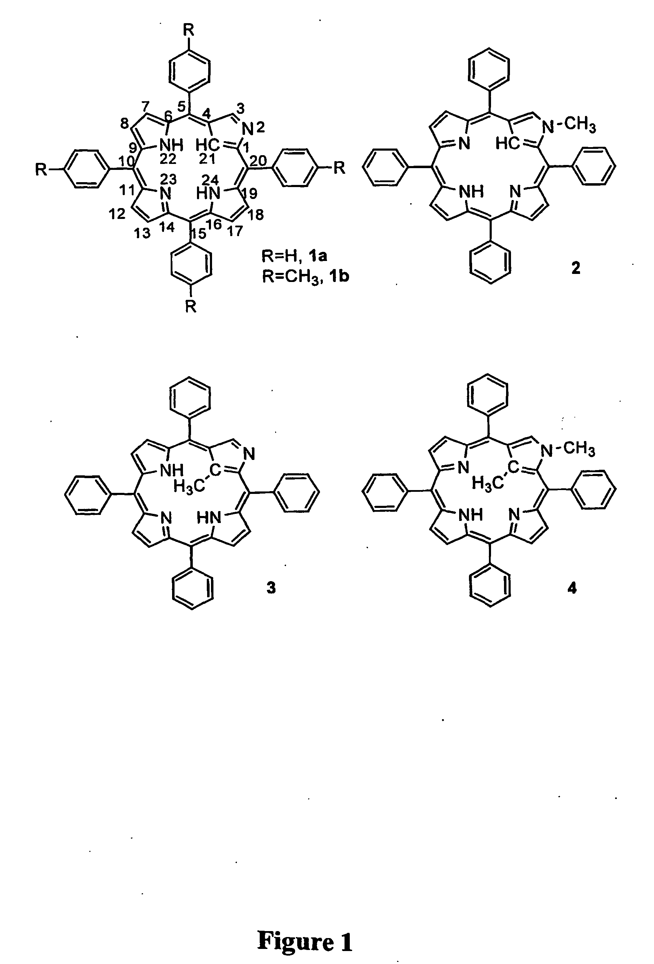

In 1994, the groups of Furuta and Latos-Grazynski independently reported on the synthesis ofN-confused porphyrin compounds **1a** and **1b** (see Figure 1).^{1,2} Figure 1 also shows the outer N-methylated N-confused porphyrin compound **2,** the C-methylated N-confused porphyrin compound **3,** the C,N-dimethylated N-confused porphyrin compound **4,** which, along with their nickel(II) derivatives, were prepared by Latos-Grazynski *et al.*^{3,4} These compounds are all based on the porphyrin isomer 2-aza-21-carbaporphyrin, which is also known as N-confused porphyrin, or N-inverted porphyrin.

The porphyrin isomer was of interest given its structure as a tetradentate ligand to bind metal ions and form a carbon-metal bond.² This interest led to studies on nickel(II) and copper(II) metal complexes of N-confused porphyrin and its methylated derivatives^{4,5} as well as further derivatives to form σ-phenyl or σ-alkyl coordinated groups on the metal group in the metal-carbon bond.⁶

Geier *et al.* describe a one flask method of preparing N-confused tetraphenylporphyrin by use of methanesulfonic acid (MSA).⁷ They further show the affects of time, MSA concentration, reactant concentration, and order of contact with reactants on the yield of N-confused tetraphenylporphyrin and tetraphenylporphyrin. For production of N-confused tetraphenylporphyrin, tetraphenylporphyrin may be viewed as a contaminant to be minimized from occurring in the synthesis of N-confused tetraphenylporphyrin.

### Disclosure of the Invention

The present invention, however, is directed to N,N'-dimethylated N-confused porphyrins which comprise both a outer ring methyl group and an inner methyl group on one of the three available positions: 22-N, 23-N, and 24-N and their use.

The present invention provides for novel therapeutic macrocycle compounds useful in photodynamic therapy that are based on the N-canfused porphyrin ring system. The macrocycle compounds contain at least two N-bonded methyl groups (hence they are at least "N,N'-dimethylated) and are optionally substituted at the *meso* positions. Compounds of the invention have an absorption spectra that makes them suitable for use in therapeutic or industrial applications, including treatment of human beings and animals or application in agricultural or commercial processes.

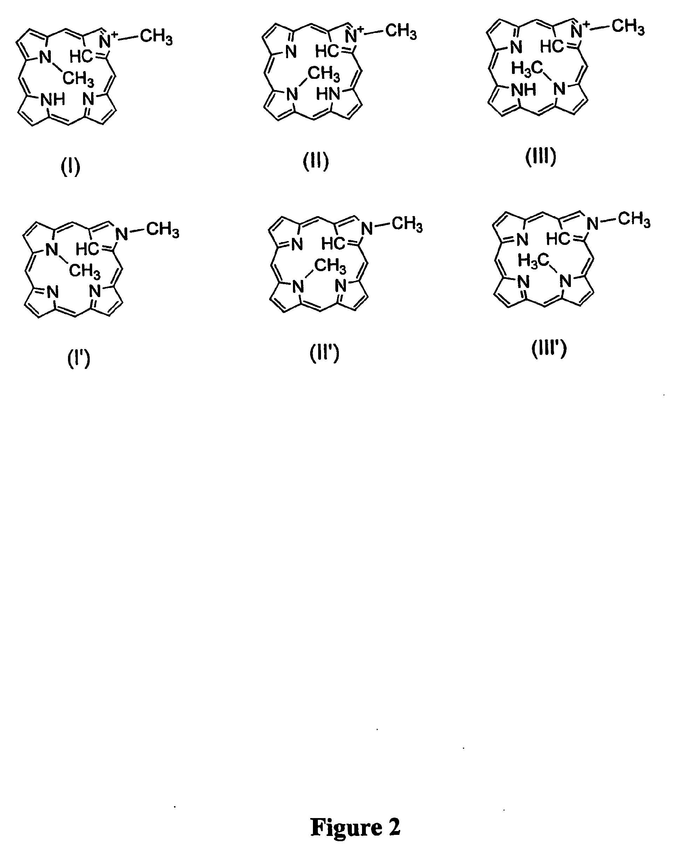

Exemplary compounds of the invention are salts of molecules having the formulas shown in Figure 2. As shown therein, the compounds have a single outer ring N-metbyl group as well as an inner N-methyl group to form a N,N'-dimethylated N-confused porphyrin. While the strictures shown in Figure 2 represent different isomeric forms of the macrocycles of the invention, other possible isomeric forms are of course also encompassed by the invention.

Other outer ring positions of the disclosed compounds may also be modified with one or more substituents. Possible positions for modification include, but are not limited to, outer ring atoms at positions 3, 7, 8,12,13,17, or 18.

Suitable methods of preparing the compounds of the invention are also provided. The methods may be considered as being composed of sequential reactions, beginning with the preparation of N-confused porphyrins by reactions known in the art. The inversion of one of the pyrrole rings in the resulting macrocycle gives rise to the outer ring N group. The remainder of the synthetic method may be considered a methylation reaction of the outer ring N group along with an inner ring N group with any suitable methylating agent.

The invention also use of the compounds of the invention in preparation of a medicament for photodynamic therapy for the treatment of various conditions, tissues and cells of a subject in need thereof. Such uses are based upon the ability of the disclosed compounds to generate singlet oxygen upon activation with irradiation containing at least one wavelength absorbed by a compound of the invention.

### Brief Description of the Drawings

Figure 1 shows the structures of various N-confused porphyrins and their singly and doubly methylated forms: N-confused tetraphenylporphyrin (CTPPH₂, **1a**); N-confused tetra ((ρ-tolyl)porphyrin **1b**); 2-aza-2-methyl-5,10,15,20-tetraphenyl-21-carbaporphyrin (2-NCH₃CTPPH₂, **2**); 2-aza-21-methyl-5,10,15,20-tetraphenyl-21-carbaporphyrin (**3**); and 2-aza-2,21-dimethyl-5,10,15,20-tetraphenyl-21-carbaporphyrin (**4**).

Figure 2 shows exemplary structures of the compounds of the invention. Each formula pair (I and I', II and II', and III and III') are directed to different N,N'-dimethylated N-confused porphyrins. I, II and III are isomers of porphyrin salts while I', II' and III' are isomers of free base porphyrins. The numbering of the positions around the N-confused porphyrin macrocycle are as set forth above for structures 1a and 1b. The meso positions are those with respect to the carbon atoms at positions 5, 10, 15, and 20.

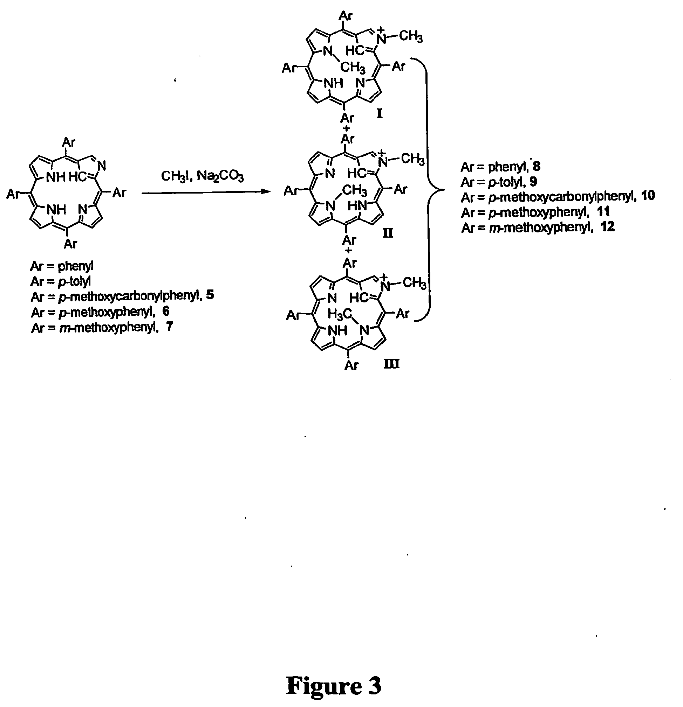

Figure 3 shows an exemplary methylation reaction for the preparation of a series of N,N'-dimethylated N-confused porphyrins of the invention: N,N'-dimethylated 2-aza-5,10,15,20-tetraphenyl-21-carba-porphyrin-HI (**8**), N,N'-dimethylated 2-aza-5,10,15,20-tetra(*p*-tolyl)-21-carba-porphyrin·HI (**9**), N,N'-dimethylated 2-aza-5,10,15,20-tetra(*p*-methoxycarbonylphenyl)-21-carba-porphyrin·HI (**10**), N,N'-dimethylated 2-aza-5,10,15,20-tetra(*p*-methoxyphenyl)-21-carba-porphyrin·HI (**11**) and N,N'-dimethylated 2-aza-5,10,15,20-tetra(*m*-methoxyphenyl)-21-carba-porphyrin·HI (**12**). The three possible isomers are shown vertically and may be designated the **"I, II,** and **III"** isomers.

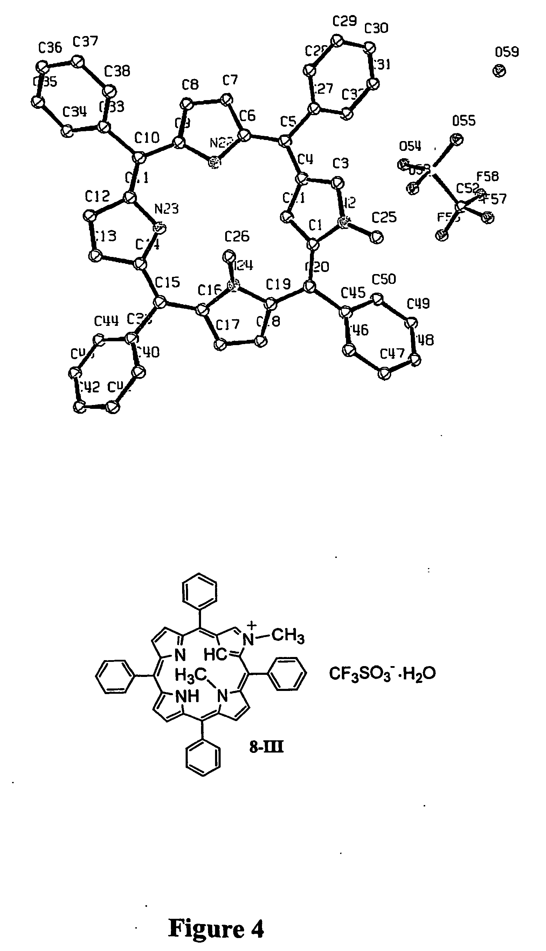

Figure 4 shows two representations of the **8-III** isomer (2-aza-2,24-dimethyl-5,10,15,20-tetraphenyl-21-carba-porphyrin) from Figure 3 as a salt with CF₃SO₃⁻˙H₂O. The first representation is an ORTEP (Oak Ridge Thermal Ellipsoid Plot) drawing of compound **8-III** showing atomic labeling and thermal ellipsoids at the 30% probability level. Compound **8-III** was prepared by exchange of CF₃SO₃⁻˙H₂O for the I⁻ salt form of compound **8-III** from the reaction scheme of Figure 3. The structure of **8-III** has been determined by X-ray crystallography, which confirmed that the porphyrin is N,N'-dimethylated.

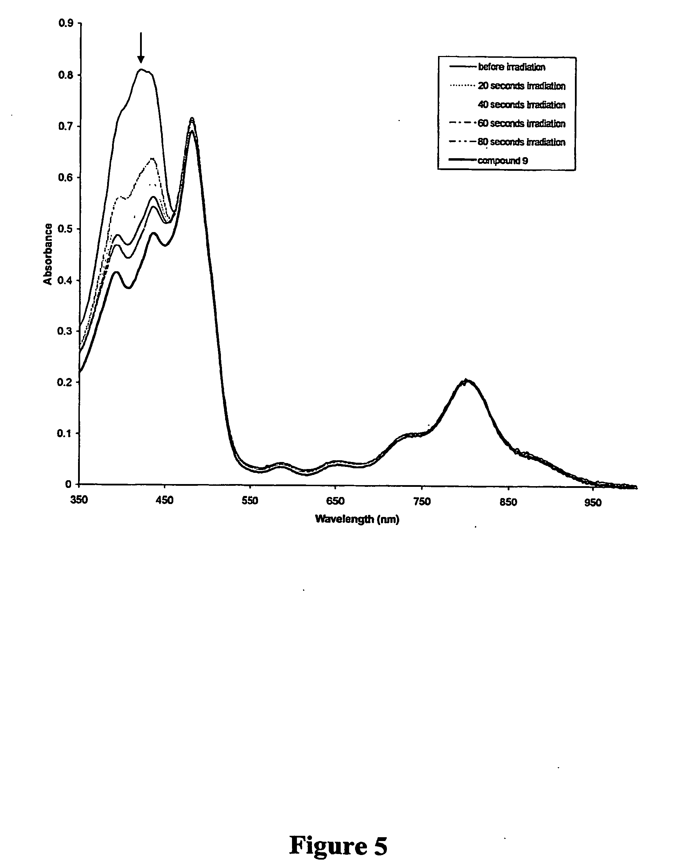

Figure 5 shows a UV-vis spectra of a solution of DPBF and compound **9** before and after irradiation.

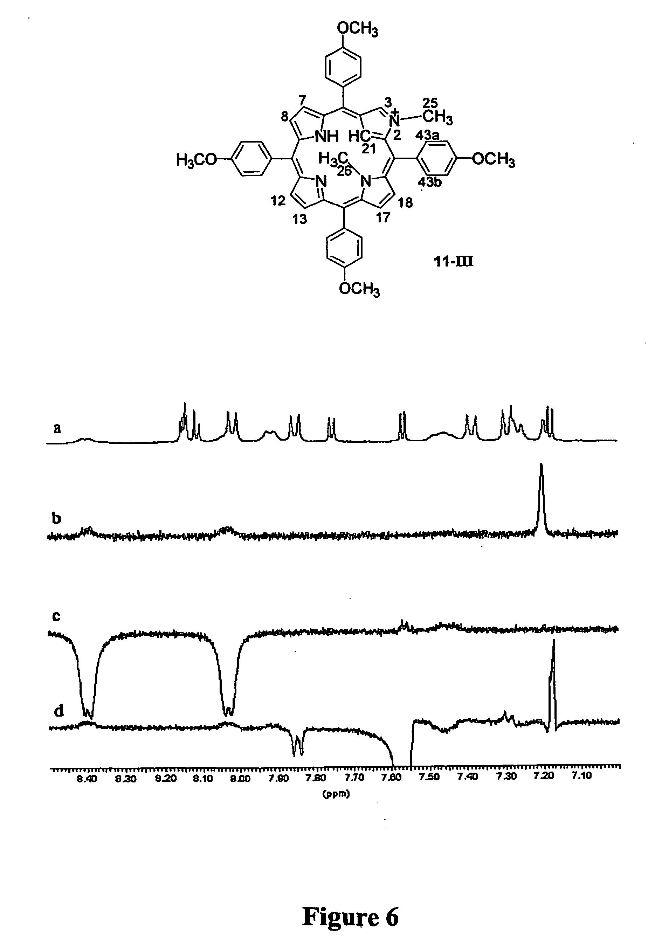

Figure 6 shows the formula and ¹H NMR spectra of compound **11-III** in CD₂Cl₂: (a) no irradiation; (b) upon irradiation at 3.81 ppm; (c) upon irradiation at 8.40 ppm; (d) upon irradiation at 7.56 ppm.

### Modes of Carrying Out the Invention

Macrocycle compounds useful in photodynamic therapy and based on the N-confused porphyrin ring system are disclosed herein. In one aspect, the invention provides a group of macrocycle compounds containing two N-bonded methyl groups represented by the following formulas:

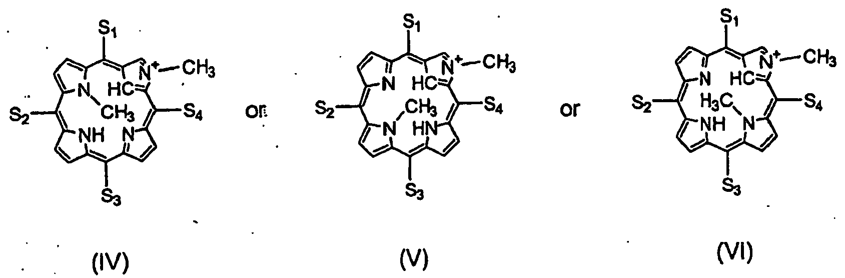

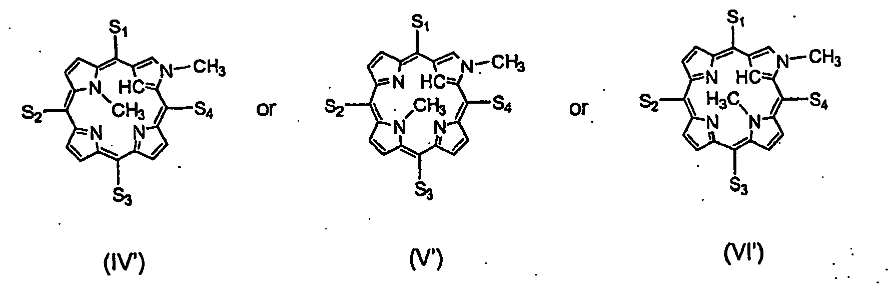

S¹ through S⁴ are identical or different and are individually selected from H or a group comprising any one of a large number of substituted or unsubstituted alkyl groups, substituted or unsubstituted cycloalkyl groups, substituted or unsubstituted aryl rings, substituted or unsubstituted aromatic rings, substituted or unsubstituted heterocyclic rings, wherein the substitution is selected from a halogen atom; thiol; a carbonyl group; a primary, secondary, tertiary or quaternary amino group; nitrile; a phosphate group and a sulfonate group. When one or more of S¹ through S⁴ is an alkyl group, it preferably has from 1 to 18 carbon atoms, more preferably 1 to 12 carbon atoms and, even more preferably, 1-6 carbon atoms. Non-limiting examples of typical alkyl groups are methyl, ethyl, isopropyl, sec-butyl, tert-butyl, n-pentyl and n-octyl.

When one or more of S¹ through S⁴ is an alkyl group, it may be unsubstituted or , substituted with a halogen atom, such as fluorine, chlorine or bromine; thiol; a carbonyl group, such as when the alkyl group is an aldehyde, ketone, carboxylic acid (e.g., a fatty acid) or ester or amide; a primary, secondary, tertiary, or quaternary amino group; nitrile; a phosphate group; a sulfonate group; or other similar groups.

When one or more of S¹ through S⁴ is a cycloalkyl group, it preferably contains from 3 to 7 carbon atoms. Non-limiting examples of typical cycloalkyl groups include cyclopropyl, cyclohexyl, and cycloheteroalkyl, such as glucopyranose or fructofuranose sugars. When one or more of S¹ through S⁴ is a cycloalkyl group, it may be unsubstituted or substituted with any of the same substituents described above for the case when one or more of S¹ through S⁴ is an alkyl group.

When one or more of S¹ through S⁴ is an aryl group, it preferably contains from 5 to 12 carbon atoms, optionally containing one or more rings that are fused to the existing conjugated porphyrin ring structure. Non-limiting examples of suitable aromatic rings include phenyl, naphthyl, and anthracenyl.

Non-limiting examples of heterocyclic rings for use in the present invention include furan, thiophene, pyrrole, isopyrrole, 3-isopyrrole, pyrazole, 2-isoimidazole, 1,2,3-triazole, 1,2,4-triazole, 1,2-dithiole, 1,3-dithiole, 1,2,3-oxathiole, isoxazole, oxazole, thiazole, isothiazole, 1,2,3-oxadiazole,1,2,4-oxadiazole,1,2,5-oxadiazole, 1,3,4-oxadiazole, 1,2,3,4-oxatriazole, 1,2,3,5-oxatriazole, 1,2,3-dioxazole, 1,2,4-dioxazole, 1,3,2-dioxazole, 1,3,4-dioxazole, 1,2,5-oxathiazole, 1,3-oxathiole, benzene, 1,2-pyran. 1,4-pyran, 1,2-pyrone, 1,4-pyrone, 1,2-dioxin, 1,3-dioxin, pyridine, N-alkyl pyridinium, pyridazine, pyrimidine, pyrazine, 1,3,5-triazine, 1,2,4-tnazine, 1,2,3-triazine, 1,2,4-oxazine, 1,3,2-oxazine, 1,3,6-oxazine, 1,4-oxazine, o-isoxazine, p-isoxazine,1,2,5-oxathiazine, 1,4-oxazine, o-isoxazine, p-isoxazine, 1,2,5-oxathiazine, 1,2,6-oxathiazine, 1,4,2-oxadiazine, 1,3,5,2-oxadiazine, azepine, oxepin, thiepin, 1,2,4-diazepine, indene, isoindene, benzofuran, isobenzofuran, thionaphthene, isothionaphthene, indole, indolenine, 2-isobenzazole, 1,4-pyrindine, pyrando[3,4-b] pyrrole, isoindazole, indoxazine, benzoxazole, anthranil, naphthalene, 1,2-benzopytan, 1,2-beozopyrone, 1,4-benzopyrone, 2,1-benaopyrone, 2,3-benzopyrone, quinoline, isoquinoline, 1,2-benzodiazine, 1,3-benzodiazine, naphthyridine, pyrido[3,4-b]-pyridine, pyrido[3,2-b-]-pyridine, pyrido[4,3-b]-pyridine, 1,3,2-benzoxazine, 1,4,2-benzoxazine, 2,3,1-benzoxazine, 3,1,4-benzoxazine, 1,2-benzisoxazine, 1,4-benzisoxazine, anthracene, phenanthrene, carbazole, xanthene, acridine, and purine.

Another embodiment of the invention relates to N,N'-dimethylated N-confused porphyrin represented by any one of formulas IV", IV"', V", V"', VI" or VI"' as shown below 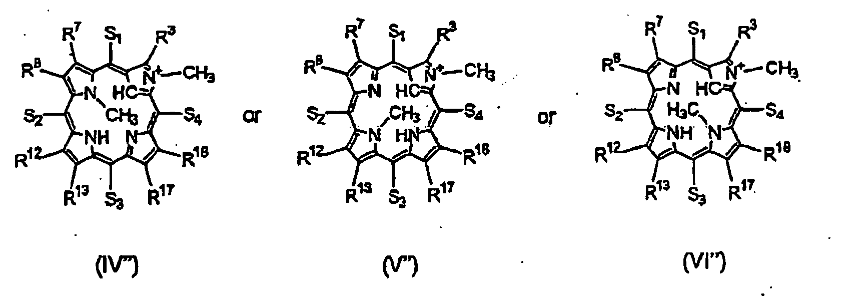 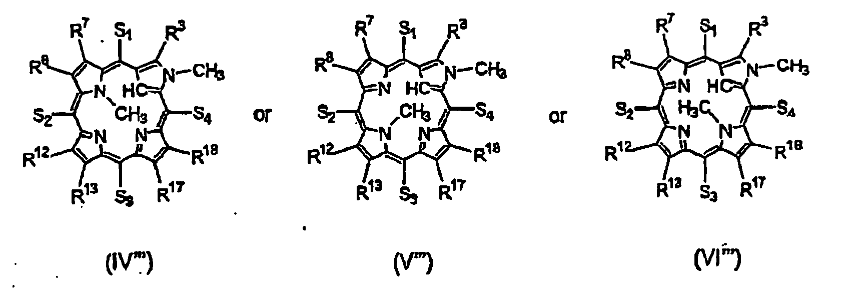 wherein said compound is labeled or in a salt form;
S¹ through S⁴ are individually selected from H or a group comprising a substituted or unsubstituted alkyl group, cycloalkyl group, aryl ring, aromatic ring or heterocyclic ring; are represented by the structure: 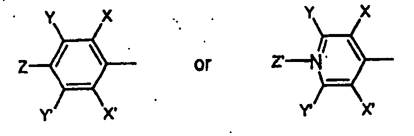 wherein X, Y, Z, X', Y' and Z' are independently selected from hydrogen, halogen, C₁₋₅ alkyl, C₁₋₅ alkoxy, hydroxy, carboxylic acid or acid salt, carboxylic acid ester, sulfonic acid or acid salt, sulfonic acid ester, phosphoric acid, phosphato or phosphate ester, amino, cyano, nitro, wherein, when one or more of S₁ to S₄ is a substituted group, the substitution is selected from a halogen atom; thiol; a carbonyl group; a primary, secondary, tertiary or quaternary amino group; nitrile; a phosphate group and a sulfonate group and R³, R⁷, R⁸, R¹², R¹³, R¹⁷, and R¹⁸ are individually selected from H or a group comprising a substituted or unsubstituted alkyl group, alkene containing group, or alkyne containing group wherein the alkyl, alkene or alkyne containing groups may be optionally and individually substituted by group selected from a halogen atom; thiol; a carbonyl group; a primary, secondary, tertiary or quaternary amino group; nitrile; a phosphate group; and a sulfonate group.
X, Y, Z, X', Y' and Z' can be any one of a large number of substituents and are generally used to "fine tune" the biological activity, the biodistribution, the absorption and clearance characteristics, and the physical properties of the desired product One way in which this may be done by selecting substituents in such a manner that a compound represented by formula (IV), (IV'), (V), (V'), (VI) or (VI') is an amphiphillic molecule. By "amphiphillic" is meant the molecule becomes more asymmetric, such as
   (1) having both (a) a highly polar, water-soluble region and (b) a highly hydrophobic, water-insoluble region;
   (2) having both (a) a nonionic region and (b) an ionic region; or
   (3) having both (a) an anionic portion and (b) a cationic portion.

However, it should be noted that the invention also includes N,N' dimethylated N-confused compounds having substantially or exactly identical aryl or heterocyclic meso-substituents. Further, any aryl or heterocyclic meso-substituent chosen should also have no adverse effect on the ability of the compound to undergo the reactions used to prepare the compounds of the invention.

X, X', Y, Y' and Z are independently (1) hydrogen; (2) halogen, such as fluoro, chloro, iodo and bromo; (3) C₁₋₅ alkyl, such as methyl, ethyl, n-propyl, isopropyl, t-butyl, n-pentyl and the like groups; (4) C₁₋₅ alkoxy, such as methoxy, ethoxy, isopropoxy, n-butoxy, t-pentoxy and the like; (5) hydroxy; (6) carboxylic acid or acid salt, such as -CH₂COOH, -CH₂COO-Na⁺, -CH₂CH(Br)COOH, -CH₂CH(CH₃)COOH, -CH(Cl)- CH₂CH(CH₃)-COOH,- CH₂CH₂-C(CH₃)₂-COOH, -CH₂CH₂-C(CH₃)₂-COO⁻K⁺,- CH₂- CH₂- CH₂- CH₂-COOH, C(CH₃)₃-COOH, CH(Cl)₂-COOH and the like; (7) carboxylic acid ester, such as -CH₂CH₂COOCH_{3.} -CH₂CH₂COOCH₂CH₃, - CH₂CH(CH₃)COOCH₂CH₃, - CH₂CH₂CH₂COOCH₂CH₂CH₃, -CH₂CH(CH₃) ₂COOCH₂CH₃, and the like; (8) sulfonic acid or acid salt, for example, group I and group II salts, ammonium salts, and organic cation salts such as alkyl and quaternary ammonium salts; (9) sulfonic acid ester; such as methyl sulfonate, ethyl sulfonate, cyclohexyl sulfonate, p-tosylate, o-tosylate and the like; (10) phosphoric acid, phosphato or phosphate ester, such as O-ethyl phosphate, O-O-diethyl phosphate, or O-ethyl phosphonic acid; (11) amino, such as unsubstituted primary amino, methylamino, ethylamino, n-propylamino, isopropylamino, 5-butylamino, sec-butylamino, dimethylamino, trimethylamino, diethylamino, triethylamino, di-n-propylamino, methylethylamino, dimethyl-sec-butylamino, 2-aminoethanoxy, ethylenediamino, 2-(N-methylamino)heptyl, cyclohexylamino, benzylamino, phenylethylamino, anilino, N-methylanilino, N,N-dimethylanilino; N-methyl-N-ethylanilino, 3,5-dibromo-4-anilino, p-toluidino, diphenylamino, 4,4'-dinitrodiphenylamino and the like; (12) cyano; (13) nitro.

In a preferred embodiment, X, X', Y, Y' and Z are independently hydrogen, halogen, C₁₋₅ alkyl, C₁₋₅ alkoxy, hydroxy, carboxylic acid or acid salt, carboxylic acid ester, sulfonic acid or acid salt, sulfonic acid ester, substituted or unsubstituted amino, cyano, nitro, and Z' is hydrogen or C₁₋₅ alkyl. In another embodiment, X, Y, X' and Y' are each hydrogen, and Z is selected from the group consisting of hydrogen, halogen, C₁₋₅ alkyl, C₁₋₅ alkoxy, hydroxy, carboxylic acid, carboxylic acid ester, sulfonic acid ester (especially aromatic sulfonic acid ester), nitro, amino (especially lower alkyl amino), cyano,

In yet another embodiment, X, Y, Z, X' and Y' are independently selected from the group consisting of hydrogen, methyl, ethyl, t-butyl, methoxy, hydroxy, OR where R is an alkyl group or a fatty acid group having from 6 to 18 carbon atoms, fluoro, chloro, iodo, bromo, -C(O)-OCH₃, cyano, nitro. In a further preferred embodiment, X, X', Y and Y' and Z are independently selected from the group consisting of hydrogen, halogen, C₁₋₅ alkyl, C₁₋₅ alkoxy, hydroxy, carboxylic acid or acid salt, carboxylic acid ester, sulfonic acid ester, sulfonic acid or acid salt, nitro, amino, cyano.

In a particularly preferred embodiment, S¹ through S⁴ are independently selected from the group consisting of phenyl, ρ-tolyl, ρ-methoxycarbonylphenyl, ρ-methoxphenyl, *m*-methoxyphenyl, naphthyl, pyridinyl, lower N-alkyl pyridinium salts, indolyl, pyrazinyl, pyrimidinyl, imidazolyl, triazolyl, pyrrolyl, pyrazolyl, pyridazinyl, indolizinyl, furanyl, and thiophenyl Even more preferably, S¹ through S⁴ are identical.

In another embodiment, the present invention includes the recognition that the 2-N-methyl group in the disclosed compounds may affect the nature of the *meso* position moieties at carbon atoms 5 and 20, and thus affect the structure of the overall compound. This is evident in the case of compounds **12**, wherein the 2-N-methyl group hinders rotation of the adjacent *m*-methoxyphenyl group. This results in compounds **12** being a mixture of atropisomers in addition to structural isomers, making it a more complex mixture. Methods for the separation of atropisomers are known in the art.

Therefore, the present invention includes structures that do not give rise to atropisomers due to inhibition of rotation of a moiety at at least one of carbon atoms 5 or **20**. This may be accomplished by the presence of a linker moiety between at least one of carbon atoms 5 or 20 and the corresponding S¹ or S⁴ group which distances the S¹ or S⁴ group sufficiently far from the 2-N-methyl group so that rotation of the group may occur freely. Preferred linker groups are short alkyl chains of 1-3 carbon atoms, although chains of up to 4, 5, or 6 carbon atoms (optionally including at least one heteroatom selected from O, N, P or S) may also be used.

In a further aspect, the invention provides macrocycle compounds represented by the following formulas:

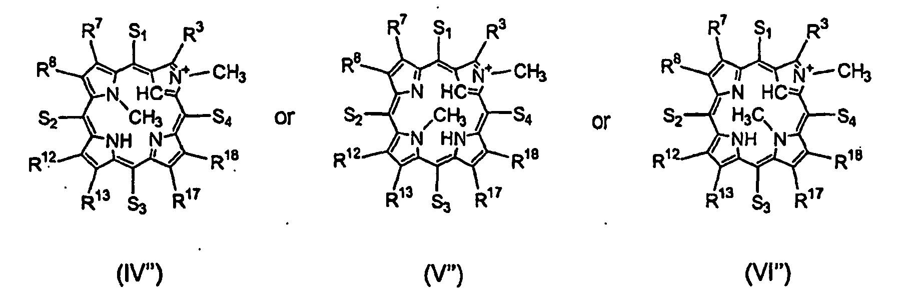

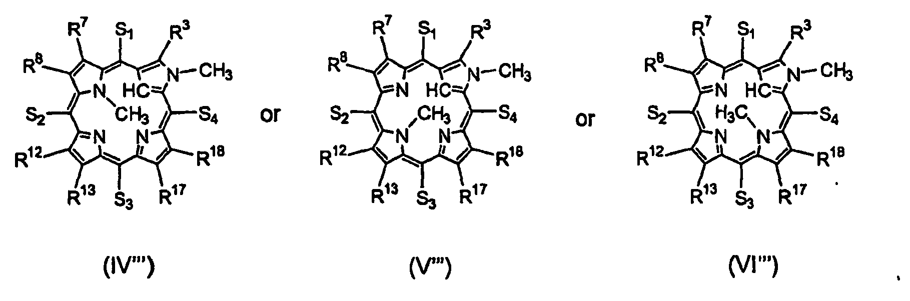

Positions S¹ through S⁴ are as defined above, while groups R³, R⁷, R⁸, R¹², R¹³, R¹⁷, and R¹⁸ (where the numbering of each R group is based on the numbering and position of atoms in the outer ring of the macrocycle) are identical or different and are individually selected from H or a group comprising any one of a large number of substituted or unsubstituted alkyl groups, alkene containing groups, or alkyne containing groups. These positions are preferably substituted by a alkyl, alkene, or alkyne containing group with from 1 to 18 carbon atoms, more preferably 1 to 12 carbon atoms and, even more preferably, 1-6 carbon atoms. Non-limiting examples of typical alkyl groups are methyl, ethyl, isopropyl, sec-butyl, tert-butyl, n-pentyl and n-octyl.

The alkyl, alkene, or alkyne containing groups may be optionally, and individually, substituted by a halogen atom, such as fluorine, chlorine or bromine; thiol; a carbonyl group, such as when the alkyl group is an aldehyde, ketone, carboxylic acid (e.g., a fatty acid) or ester or amide; a primary, secondary, tertiary, or quaternary amino group; nitrile; a phosphate group; a sulfonate group; or other similar groups.

Exemplary compounds of the invention are salts of the structures represented by the formulas herein. In addition to CF₃SO₃⁻ and I⁻ salts, non-limiting salts include F⁻, Cl⁻, Br⁻, NO₃⁻, BF₄⁻, CH₃SO₃⁻, CH₃COO⁻ and CF₃COO⁻. Such salts may also optionally be in a hydrate form comprising one or more molecules of H₂O. Preferred for the practice of the invention are pharmaceutically acceptable salts of the disclosed compounds.

### Preparation

The present invention also provides methods for the formation of N,N'-dimethylated N-confused porphyrins. The methylation of CTPPH₂ (compound **1a** in Fig. 1) with CH₃I in CH₂Cl₂ yields 2-NCH₃CTPPH₂ (compound **2** in Fig. 1).³ Without being bound by theory, it is assumed that protonation of compound **2** by HI generated from the methylation reaction prevents inner N-methylation. Therefore, and as an additional aspect of the invention, the addition of a base, such as, but not limited to, Na₂CO₃, neutralizes the intermediate 2-NCH₃CTPPH₂·HI, making the inner nitrogens more nucleophilic towards methylation and resulting in N,N'-dimethylation. The present invention thus provides quantitative dimethylation of N-confused porphyrins, as shown by TLC, was carried out in CH₂Cl₂ solution using CH₃I and Na₂CO₃. The resulting N,N'-dimethylated N-confused porphyrin salts have an intense absorption at approximately 790 nm, and therefore, are of interest for use as photosensitizers in photodynamic therapy (PDT).

Each of the N,N'-dimethylated N-confused porphyrin salts shown as compounds **8-12** in Figure 3 may have three structural isomers in theory, since there are three possible positions, 22-N, 23-N, 24-N, for inner N-methylation. The major isomers of compounds **8-11** were isolated by recrystallization and determined by X-ray diffraction analyses or NMR spectroscopy to contain an inner methyl group at the N-24 position.

While synthesis of N,N'-dimethylated N-confused porphyrin has been discussed above with respect to the use of CH₂Cl₂ solution to react a N-confused porphyrin with CH₃I and Na₂CO₃, other solvents, methylating agents, and bases known in the art may be used in the practice of the invention. Non-limiting examples of these include the following. Solvent: aromatic solvents, such as pyridine, toluene and benzene; chlorinated solvents, such as CHCl₃, dichloromethane and 1,1-dichloroethane; water, ethers, such as diethyl ether, tetrahydrofuran, diethylene glycol and glycol dimethyl ether (ethylene glycol dimethyl ether); ketones such as acetone and pinacolone; acetonitrile; DME, DMF and DMSO; and mixtures thereof. Methylating agent: methylfluorosulfonate, methyltrifluoromethanesulfonate, and dimethylsulfate. Base: triethylamine, pyridine, NaOH, and KOH. Other solvents, methylating agents, and bases known in the art, or readily determined, to be equivalent to the above may also be used in the practice of the present invention.

Preferably, the reaction occurs for about 1, about 2, or about 1-2 days in the absence of light, although low levels of light may also be used. Most preferred is reaction for about 2 days. The temperature of the reaction may vary greatly but is preferably room temperature (or about 25°C). Most preferred is the use of a reaction temperature range of about 5-6 degrees above or below 25 °C.

The salts of the compounds of the invention may be exchanged by methods known in the art. Therefore, compounds prepared as one salt (e.g. I⁻) may be converted to another salt form (e.g. CF₃SO₃⁻).

Routine procedures can be used to isolate the compounds and isomers resulting from the above methods. Non-limiting examples include extraction with any immiscible liquid, eluting on a silica gel column or other type of chromatography, drowning out in a non-solvent, precipitating out or otherwise crystallizing, evaporation of solvent, or some combination of these or other conventional methods. A preferred method of isolating the compounds of the invention is crystallization.

If further purification of the product compound(s) is/are desired, it may be subjected to additional purification procedures, such as recrystallization, eluting on a silica gel chromatography column, and combinations of these methods. Although some of the reagents may be permitted to remain with the product compound(s) without affecting the activity (such as, but not limited to photosensitivity) of the compound(s), it is preferable to isolate and/or purify the compound(s) to increase the range of uses for the compound(s).

The compounds of the invention are useful as photosensitizers used in photodynamic therapy (PDT), as well as being synthetic intermediates for making related photosensitizers. As photosensitizers, the compounds of the invention are useful in sensitizing neoplastic cells or other abnormal tissues to destruction by irradiation with visible light. Upon photoactivation, the energy of photoactivation is believed to be transferred to endogenous oxygen, thus converting it to singlet oxygen. This singlet oxygen is thought by some to be responsible for the observed cytotoxic effect. Alternatively, there may be direct electron transfer from the photoactivated molecule. The method of van Lier, Photobiological Techniques, 216, 85-98 (Valenzo *et al.* eds. 1991) can be used to confirm the ability of any given compound to generate singlet oxygen effectively, thus making it a good candidate for use in PDT.

Alternatively, the ability to generate singlet oxygen may be tested as follows. DPBF (1,3-diphenylisobenzofuran)⁸ is used to determine the ability of N,N'-dimethylated N-confused porphyrin salts to generate singlet oxygen. DPBF reacts quickly with singlet oxygen and its absorption decay around 418 nm can be easily monitored. The reaction products of DPBF have no absorption in the visible region and do not quench singlet oxygen. A solution containing DPBF and N,N'-dimethylated N-confused porphyrin salts (e.g. compound **9** in Fig. 3) was irradiated with a halogen lamp using a filter (~700 nm), and monitored by UV-vis spectroscopy at 418 nm. Substantial decay of UV-vis signal at 418 nm was observed suggesting that N,N'-dimethylated N-confused porphyrin salts generate singlet oxygen (see Figure 5).

Additionally, the photoactivated forms of porphyrin are able to fluoresce, and this fluorescence may be used in the diagnostic imaging of tissues, such as, but not limited to, the imaging of tumor or other tissue types.

### Administration and Use

The compounds of the invention may be used in a manner analogous to the use of any photosensitizer in photodynamic therapy (PDT). These include, but are not limited to, the diagnosis or treatment of cancer, the reduction of activated leukocytes, the treatment of ocular disorders, the treatment and prevention of neovasculature and angiogenesis, the destruction of viruses and cells infected thereby, the treatment of atherosclerotic plaques, the treatment of restenosis, and others. In addition, the compounds may be photoactivated by appropriate excitation wavelengths to fluoresce visibly. This fluorescence can then be used to localize a tumor or other target tissue.

Of course the compounds of the invention may be used singly or in combination with each other or other photosensitizers known in the art. Preferably, the compounds are administered in an effective amount such that a photodynamic effect sufficient to treat or prevent any of the diseases and conditions as disclosed herein may occur.

In addition to in vivo use, the compounds of the invention can be used in the treatment of materials in vitro to destroy harmful viruses or other infectious agents. For example, blood plasma or blood that is to be used for transfusion or banked for future transfusion, can be treated with the compounds of the invention and irradiated to effect sterilization. In addition, biological products such as Factor VIII, which are prepared from biological fluids, can be irradiated in the presence of the compounds of the invention to destroy contaminants.

The photosensitizers made from the compounds of the invention can be formulated into pharmaceutical compositions for administration to the subject or applied to an in vitro target using techniques generally known in the art. A summary of such pharmaceutical compositions may be found, for example, in Remington's Pharmaceutical Sciences, Mack Publishing Co., Easton, PA. The compounds of the invention can be used singly or as components of mixtures. A preferred form of the compounds is as a liposomal formulation.

Generally, the compounds of the invention, labeled or unlabeled, may be administered parenterally or by injection. Injection may be intravenous, subcutaneous, intramuscular, intrathecal, or even intraperitoneal. However, the compounds may also be administered by aerosol intranasally or intrapulmonarally, or topically. Formulations designed for timed release are also with the scope of the invention. The compounds of the invention may be labeled isotopically (e.g. with a radioisotope) or by another other means, including, but not limited to, the use of chromophores or fluorescent moieties.

Injectables can be prepared in conventional forms, either as liquid solutions or suspensions, solid forms suitable for solution or suspension in a liquid prior to injection, or as emulsions. Suitable excipients are, for example, water, saline, dextrose, glycerol and the like. Of course, these compositions may also contain minor amounts of nontoxic, auxiliary substances, such as wetting or emulsifying agents, pH buffering agents, and so forth.

Systemic administration can be implemented through implantation of a slow release or sustained release system, by suppository, or, if properly formulated, orally. Formulations for these modes of administration are well known in the art, and a summary of such methods may be found, for example, in Remington's Pharmaceutical Sciences (supra).

If treatment is to be localized, such as for the treatment of superficial tumors or skin disorders, the compound can be administered topically using standard topical compositions, such as lotions, suspensions, or pastes.

The quantity of the photosensitizer compound to be administered depends upon the choice of active ingredient, the condition to be treated, the mode of administration, the individual subject, and the judgment of the practitioner. Depending on the specificity of the preparation, smaller or larger doses may be needed. For compositions that are highly specific to target tissues, such as those with a highly specific monoclonal immunoglobulin preparation or a specific receptor ligand, dosages in the range of 0.05-1 mg/kg are suggested. For compositions that are less specific to the target tissue, larger doses, up to 1-10 mg/kg may be needed. The foregoing ranges are merely suggestive, as the number of variables in regard to an individual treatment regime is large, and considerable excursions from these recommended values are not uncommon.

For activation of a photosensitizing compound of the invention, any suitable absorption wavelength is used. This can be supplied using the various methods known to the art for mediating cytotoxicity or fluorescence emission, such as visible radiation, including incandescent or fluorescent light sources or photodiodes such as light emitting diodes. Laser light can also be used for *in situ* delivery of light to a localized photosensitizer. In a typical protocol, for example, a compound of the invention is administered prior to irradiation.

Preferably, electromagnetic radiation containing one or more wavelength absorbed by the photosensitizing compound of the invention, such as from ultraviolet to visible and infra red light, is delivered after administration of the compound, compositions and formulations of the invention. Also preferred in the invention is the use of low-dose PDT. By "low-dose PDT", it is meant a total photodynamic therapy experience at substantially lower levels of intensity than that ordinarily employed. Generally, there are three significant variables -- the concentration of the photosensitizing agent, the intensity of the radiation employed and the time of exposure to light, which determines the total amount of energy ultimately delivered to the target tissue. Generally, an increase in one of these factors permits a decrease in the others.

For example, if it is desired to irradiate only for a short period of time the energy of irradiation or the concentration of the drug may be increased. Conversely, if longer time periods of irradiation are permitted, lower irradiation intensities and lower drug concentrations are desirable. The use of low dose PDT offers an additional advantage in the form of reducing the likelihood of PDT side effects such as damage to unintended tissues.

It is understood that the manipulation of these parameters will vary according to the nature of the tissue being treated and the nature of the compound of the invention being employed. However, in general, low-dose PDT employs combinations of the drug concentration, radiation intensity, and total energy values which are several fold lower than those conventionally used for destroying target tissues such as tumors and unwanted neovascularization. One measure might be the product of photosensitizing compound concentration (e.g., in ng/ml) x intensity (e.g., in mW/cm²) x time (e.g., in seconds). However, it is difficult to set absolute numbers for this product since there are constraints on each of the parameters individually. For example, if the intensity is too low, the compound will not be photoactivated consistently; if the intensity is too high, hyperthermic and other damaging effects may occur. Additionally, in some instances, ambient or environmental light available at the target cell or tissue undergoing PDT may be sufficient in the absence of additional deliberate irradiation.

Similarly, concentrations of the compound(s) of the invention cannot vary over any arbitrary range. There may also be constraints on the time during which radiation can be administered. Accordingly, the product of the foregoing equation is only a rough measure. However, this approach may provide a convenient index that can be adjusted according to the relative potency of the compound employed, and in general, an increase in intensity would permit a decrease in time of irradiation, and so forth.

Having now generally described the invention, the same will be more readily understood through reference to the following examples which are provided by way of illustration, and are not intended to be limiting of the present invention, unless specified.

### Example 1

### General experimental materials and methods

Pyrrole (Acros) was distilled from CaH₂ before use. The silica gel was 230-400 mesh Silicycle). Activity III basic alumina was obtained by adding 6% water to activity I Brockman basic alumina, 60-325 mesh (Fisher). All other materials and solvents were used as received. The NMR spectra were recorded on a Bruker WH-400 or a Bruker AV-400 in the solvents indicated and were referenced to residual solvent peaks. Elemental analyses were performed on a Carlo Erba Elemental Analyzer1108. The UV-vis spectra were measured on a Cary 50. Mass spectra were determined on a KRATOS Concept IIHQ hybrid mass spectrometer. Irradiations were carried with a 250 W Osram HLX 64655 arc lamp in an Oriel lamp housing (model 66184). The light output passed through a glass filter: P70-700-S-Corion.

N-confused porphyrins were synthesized using Lindsey's procedure.⁷ To a solution of pyrrole (0.52 mL, 7.5 mmol) and arylaldehyde (7.5 mmol) in CH₂Cl₂ (750 mL) was added methanesulfonic acid (MSA) (0.34 mL, 5.2 mmol). The mixture was stirred for 30 min after which DDQ, or 2,3-dichloro-5,6-dicyanobenzoquinone, (1.50 g, 6.6 mmol) was added. After 1 min, triethylamine (1.5 mL) was added. The crude reaction mixture was chromatographed with silica gel (14 × 4.4 cm) under vacuum and eluted with CH₂Cl₂. CH₂Cl₂/ 1.2% methanol eluted the product with impurities. The fractions were collected and concentrated under vacuum and then absorbed onto 7.5 g of activity III basic alumina. The absorbed sample was added to the top of a column with 150 g activity III basic alumina in 2:1 hexanes/CH₂Cl₂. The polarity of the eluant was increased from 2:1 to 1:1 to 1:2 hexanes/CH₂Cl₂, the N-confused porphyrins were eluted with 1:2 hexanes/CH₂Cl₂ (in the case of compound **5** and **6**, Fig. 1, the polarity of the eluant was increased from CH₂Cl₂ to CH₂Cl₂/ 0.2% methanol to elute the product). The solvent was removed under vacuum and the residue was triturated with CH₂Cl₂/hexanes to yield the product. Compound **1a,** yield: 373 mg (32%); compound **1b,** yield: 274 mg (22%); compound 5, yield: 259 mg (16%); compound 6, yield: 208 mg (15%); compound **7**, yield: 316 mg (23%). See Figure 1 for structures.

### Example 2

### General method to prepare N,N'-dimethylated N-confused porphyrins

N-confused porphyrin (100 mg) was dissolved in a minimal amount of CH₂Cl₂ (about 10 mL). To this solution, CH₃I (8 mL) and Na₂CO₃ (250 mg) were added. The mixture was stirred for 2 days in the absence of light, then filtered through Celite. The filtrate was evaporated to dryness under vacuum and the residue was triturated with CH₂Cl₂/hexanes to yield the products.

### Example 3

### Singlet oxygen tests

A solution containing DPBF and N,N'-dimethylated N-confused porphyrin salts (one of compounds **8-12** or the major isomer of compounds **8-11**, see Figure 3) (OD = 0.8 - 1.0 at 418 nm, OD = 0.1 - 0.2 at irradiation wavelength) was prepared and UV-vis spectra were taken. The solution was then irradiated with a halogen lamp using a cutoff filter (~700 nm) for four 20 seconds intervals and UV-vis spectra were taken after each interval. Substantial decay of UV-vis signal around 418 nm was observed in each case. No change in UV-vis spectra was observed after a sample containing DPBF and N,N'-dimethylated N-confused porphyrin salts was left in the dark for 10 min, and there is also no change in UV-vis spectra after irradiating a solution containing only DPBF or N,N'-dimethylated N-confused porphyrin salts for 1 min. These tests indicate that N,N'-dimethylated N-confused porphyrin salts generate singlet oxygen when irradiated.

### Example 4

### Data for compounds of the invention

**Crystal Structure of 2-aza-2,24-dimethyl-5,10,15,20-tetraphenyl-21-carbaporphyrin·HCF₃SO₃ (compound 8-III in Fig 4). 8-III** (Figure 4) is the major isomer of N,N'-dimethylated 2-aza-5,10,15,20-tetraphenyl-21-carba-porphyrin-HI (see compound **8** in Figure 3). Of note, the I- was exchanged for CF₃SO₃- in **8-III.** The structure of **8-III** has been determined by X-ray crystallography, which confirms that the porphyrin is N,N'-dimethylated.

Compounds **8** (86 mg) were dissolved in 20 mL of CH₂Cl₂. Silver triflate (1.3 g) was added and the solution was stirred for 2 h. The mixture was chromatographed through a silica gel column (10 × 2.5 cm) and eluted with CH₂Cl₂ under vacuum. CH₂Cl₂ /1% CH₃OH eluted the porphyrin triflate salts. The compound was recrystallized three times with CH₂Cl₂/hexanes giving **8-III** (29 mg). Crystals of **8-III** were obtained by solvent diffusion of hexanes into a CH₂Cl₂ solution of **8-III.** R*_{f}* (silica-CH₂Cl₂/5% CH₃OH/2% Et₃N) 0.36; ¹H-NMR (400 MHz, CD₂Cl₂) δ= -1.60 (s, 1H), -1.49 (s, 3H), 3.81 (s, 3H), 7.24 (d, *J=* 5.0 Hz, 1H), 7.43 (s, 1H), 7.62 (d, *J*= 5.0 Hz, 1H), 7.70-8.13 (m, 20H), 8.16 (d, *J*= 5.1 Hz, 1H), 8.20 (d, *J*= 5.1 Hz, 1H), 8.33 (d, *J*= 4.8 Hz, 1H), 8.47 (d, *J*= 6.4 Hz, 1H); UV-vis (CH₂Cl₂) λₘₐₓ/nm (log ε) 385 (sh), 475 (4.85), 580 (3.74), 650 (3.67), 724 (sh), 790 (4.41); MS (LSIMS) 643 (M⁺, 100%); Anal. calcd for C₄₆H₃₅N₄·CF₃SO₃·1.5H₂O: C, 68.85; H, 4.67; N, 6.83; S, 3.91. Found: C, 68.99; H, 4.61; N, 6.78; S, 4.00. Crystal Data for **8-III:** C₄₇H₃₅N₄SO₃F₃ H₂O, M = 810.89, triclinic, *a* = 11.922(2) Å, *b* = 13.505(2) Å, *c* = 15.090(3) Å, α = 110.043(6)°, β = 94.106(6)°, γ = 113.859(7)°, V = 2023.9(6) Å³, T = 198.2 K, space group Pi (#2), Z = 2, µ(Mo-K_{α}) = 1.44 cm⁻¹, 11514 reflections measured, 5815 unique (R*ᵢₙₜ* = 0.056) which were used in all calculations. The final Rw(F²) was 0.176 (all data).

**2-Aza-5,10,15,20-tetra(*p*-methoxycarbonylphenyl)-21-carbaporphyrin (compound 5 in Fig 1).** R*_{f}* (silica-CH₂Cl₂/5% CH₃OH/2% Et₃N) 0.46; ¹H-NMR (400 MHz, CD₂Cl₂) δ= -5.24 (s, 1 H), -2.56 (br s, 2H), 4.08 (d, 12H), 8.18-8.58 (m, 19H), 8.60 (d, *J*= 4.7 Hz, 1H), 8.70 (s, 1H), 8.84(d, *J*= 4.7 Hz, 1H), 8.94 (d, *J*= 4.7 Hz, 1H); UV-vis (CH₂Cl₂) λₘₐₓ/nm (log ε) 444 (5.31), 544 (4.07), 586 (4.22), 728 (4.15); MS (LSIMS) 847 (MH⁺, 100%); HRMS (LSIMS) *m*/*e* calcd for C₅₂H₃₉N₄O₈: 847.27679, found 847.27667 (MH⁺); Anal. calcd for C₅₂H₃₈N₄O₈: C, 73.75; H, 4.52; N, 6.62. Found: C, 73.95; H, 4.49; N, 6.72.

**2-Aza-5,10,15,20-tetra(*p*-methoxyphenyl)-21-carba-porphyrin (compound 6 in Fig. 1).** R*_{f}*(silica-CH₂Cl₂/5% CH₃OH/2% Et₃N) 0.38; ¹H-NMR (400 MHz, CD₂Cl₂) δ= -4.92 (s, 1H), -2.31 (br s, 2H), 4.04 (s, 3H), 4.07 (s, 6H), 4.09 (s, 3H), 7.25-7.49 (m, 8H), 8.00-8.13(m, 4H), 8.25 (d, *J*= 8.6 Hz, 4H), 8.55(d, *J*= 5.2 Hz, 3H), 8.61 (d, *J*= 4.7 Hz, 1H), 8.64 (s, 1H), 8.89 (d, *J*= 4.4 Hz, 1H), 8.97 (d, *J=* 4.7 Hz, 1H); UV-vis (CH₂Cl₂) λₘₐₓ/nm (log ε) 442 (5.38), 514 (sh), 548 (sh), 592 (4.32), 736 (4.24); MS (LSIMS) 735 (MH⁺, 100%); Anal. calcd for C₄₈H₃₈N₄O₄: C, 78.45; H, 5.21; N, 7.62. Found: C, 78.11; H, 5.10; N, 7.71.

**2-Aza-5,10,15,20-tetra(*m*-methoxyphenyl)-21-carba-porphyrin (compound 7 in Fig. 1).** R*_{f}*(silica-CH₂Cl₂/5% CH₃OH/2% Et₃N) 0.50; ¹H-NMR (400 MHz, CD₂Cl₂) δ= -5.12 (s, 1H), -2.45 (br s, 2H), 3.98 (s, 6H), 4.02 (s, 3H), 4.06 (s, 3H), 7.22-7.45 (m, 4H), 7.58-7.82 (m, 8H), 7.82-8.00 (m, 4H), 8.55-8.66 (m, 3H), 8.68 (d, *J*= 4.7 Hz, 1H), 8.78 (s, 1H), 8.96(d, *J*= 4.7 Hz, 1H), 9.05 (d, *J*= 5.2 Hz, 1H); UV-vis (CH₂Cl₂) λₘₐₓ/nm (log ε) 440 (5.34), 540 (4.06), 582 (4.13), 726 (4.11); MS (LSIMS) 735 (MH⁺, 100%); HRMS (LSIMS) *m*/*e* calcd for C₄₈H₃₉N₄O₄: 735.29713, found 735.29827 (MH⁺); Anal. calcd for C₄₈H₃₈N₄O₄: C, 78.45; H, 5.21; N, 7.62. Found: C, 78.44; H, 5.18; N, 7.71.

**N,N'-Dimethylated 2-aza-5,10,15,20-tetraphenyl-21-carba-porphyrin-HI (compound 8 in Fig. 3).** Yield: 110 mg (88%). R*_{f}*(silica-CH₂Cl₂/5% CH₃OH/2% Et₃N) 0.36; ¹H-NMR (400 MHz, CD₂Cl₂) δ= -1.62 (d, *J*= 1.4 Hz, 0.65H), -1.57 (d, *J*=1.5 Hz, 0.35H), -1.48 (s, 3×0.65H), -1.39 (s, 3×0.35H), 3.62 (s, 3×0.35H), 3.82 (s, 3×0.65H), 7.18-8.54 (m, 27H); UV-vis (CH₂Cl₂) λₘₐₓ/nm (log ε) 382 (sh), 476 (4.85), 582 (3.79), 650 (3.73), 724 (sh), 788 (4.39); MS (LSIMS) 643 (M⁺, 100%); HRMS (LSIMS) *mle* calcd for C₄₆H₃₅N₄: 643.28617, found 643.28623 (M⁺); Anal. calcd for C₄₆H₃₄N₄·HI·1.5H₂O: C, 69.26; H, 4.80; N, 7.02; 1,15.91. Found: C, 68.96; H, 4.76; N, 6.86; I, 15.82.

**N,N'-Dimethylated 2-aza-5,10,15,20-tetra(p-tolyl)-21-carba-porphyrin-HI (compound 9 in Fig. 3).** Yield: 105 mg (85%). R*_{f}*(silica-CH₂Cl₂/5% CH₃OH/2% Et₃N) 0.36; ¹H-NMR (400 MHz, CD₂Cl₂) δ= -1.53 (d, *J*= 1.7 Hz, 0.65H), -1.48 (s, 3×0.65H), - 1.45 (d, *J*=1.7 Hz, 0.35H), -1.34 (s, 3×0.35H), 2.65 (m, 12H), 3.61 (s, 3×0.35H), 3.81 (s, 3×0.65H), 7.1-8.5 (m, 23H); UV-vis (CH₂Cl₂) λₘₐₓ/nm (log ε) 392 (sh), 478 (4.85), 588 (3.64), 646 (3.69), 798 (4.22); MS (LSIMS) 699 (M⁺, 100%); HRMS (LSIMS) *m*/*e* calcd for C₅₀H₄₃N₄: 699.34877, found 699.34859 (M⁺); Anal. calcd for C₅₀H₄₃N₄·I·0.5H₂O: C, 71.85; H, 5.31; N, 6.70. Found: C, 71.85; H, 5.41; N, 6.70.

**2-Aza-2,24-dimethyl-5,10,15,20-tetra(*p*-tolyl)-21-carbaporphyrin-HI (compound 9-III in Fig. 3).** Compounds 9 (109 mg) was recrystallized three times with CH₂Cl₂/hexanes to give the major isomer (34 mg). R*_{f}*(silica-CH₂Cl₂/5% CH₃OH/2% Et₃N) 0.36; ¹H-NMR (400 MHz, CD₂Cl₂) δ=-1.55 (d, *J=* 1.7 Hz, 1H), -1.49 (s, 3H), 2.63 (s, 6H), 2.66 (s, 6H), 3.82 (s, 3H), 7.22 (d, *J=* 5.2 Hz, 1H), 7.33 (d, *J*= 1.3 Hz, 1 H), 7.51-8.05 (m, 17H), 8.15 (d, *J*= 5.2 Hz, 1H), 8.18 (d, *J*= 5.2 Hz, 1H), 8.29 (d, *J=* 4.7 Hz, 1H), 8.34 (b s, 1H); UV-vis (CH₂Cl₂) λₘₐₓ/nm (log ε) 390 (sh), 435 (sh), 480 (4.78), 585 (3.54), 650 (3.56), 800 (4.26); MS (LSIMS) 699 (M⁺, 100%); Anal. calcd for C₅₀H₄₃N₄·I·H₂O: C, 71.08; H, 5.37; N, 6.63; I, 15.02. Found: C, 71.05; H, 5.31; N, 6.58; I, 14.95.

**N,N'-Dimethylated 2-aza-5,10,15,20-tetra(*p*-methoxycarbonylphenyl)-21-carba-porphyrin·HI (compound 10 in Fig. 3).** Yield: 103 mg (87%). R*_{f}*(silica-CH₂Cl₂/5% CH₃OH/2% Et₃N) 0.36; ¹H-NMR (400 MHz, CD₂Cl₂) δ= -1.79 (m, 1H), - 1.49 (m, 3H), 3.65 (s, 3×0.27H), 3.87 (s, 3×0.73H), 4.05 (m, 12H), 7.20-8.73 (m, 23H); UV-vis (CH₂Cl₂) λₘₐₓ/nm (log e) 386 (sh), 478 (4.84), 576 (3.86), 652 (3.81), 726 (sh), 792 (4.24); MS (LSIMS) 875 (M⁺, 100%); HRMS (LSIMS) *m*/*e* calcd for C₅₄H₄₃N₄O₈: 875.30809, found 875.30815 (M⁺); Anal. calcd for C₅₄H₄₃N₄O₈·I·2.5H₂O: C, 61.89; H, 4.62; N, 5.35. Found: C, 61.99; H, 4.42; N, 5.20.

**2-Aza-2,24-dimethyl-5,10,15,20-tetra(*p*-methoxycarbonylphenyl)-21-carbaporphyrin·HI (compound 10-III in Fig. 3).** Compounds **10** (78.5 mg) was recrystallized three times with CH₂Cl₂/hexanes to give the major isomer (29 mg). R*_{f}* (silica-CH₂Cl₂/5% CH₃OH/2% Et₃N) 0.36; ¹H-NMR (400 MHz, CDCl₃) δ=-1.45 (d, 4H), 3.91 (s, 3H), 4.06 (s, 12H), 7.19 (s, 1H), 7.52 (s, 2H), 7.82-8.85 (m, 20H); UV-vis (CH₂Cl₂) λₘₐₓ/nm (log ε) 385 (sh), 480 (4.83), 585 (3.67), 655 (3.16), 725 (sh), 790 (4.26); MS (LSIMS) 875 (M⁺, 100%); Anal. calcd for C₅₄H₄₃N₄O₈·I·H₂O: C, 63.53; H, 4.44; N, 5.49; I, 12.43. Found: C, 63.26; H, 4.55; N, 5.48; I, 12.20.

**N,N'-Dimethylated 2-aza-5,10,15,20-tetra(*p*-methoxyphenyl)-21-carbaporphyrin·HI (compound 11 in Fig. 3).** Yield: 112 mg (92%). R*_{f}*(silica-CH₂Cl₂/5% CH₃OH/2% Et₃N) 0.36; ¹H-NMR (400 MHz, CD₂Cl₂) δ= -1.43 (s, 3×0.7H), -1.37 (d, *J=* 1.5 Hz, 1×0.7H),), -1.22 (d, 4×0.3H), 3.60 (s, 3×0.3H), 3.82 (s, 3×0.7H), 4.00-4.14 (m, 12H), 7.07-8.50 (m, 23H); UV-vis (CH₂Cl₂) λₘₐₓ/nm (log ε) 436 (4.82), 486 (4.97), 592 (3.62), 662 (3.82), 818 (4.40); MS (LSIMS) 763 (M⁺, 100%); Anal. calcd for C₅₀H₄₃N₄O₄·I·1.5H₂O: C, 65.43; H, 5.05; N, 6.10. Found: C, 65.67; H, 4.88; N, 5.98.

**2-Aza-2,24-dimethyl-5,10,15,20-tetra(*p*-methoxyphenyl)-21-carbaporphyrin·HI (11-III) (compound 11a in Fig. 3).** Compounds 11 (89 mg) was recrystallized three times with CH₂Cl₂/hexanes to give the major isomer **11-III** (37 mg). R*_{f}*(silica-CH₂Cl₂/5% CH₃OH/2% Et₃N) 0.36; ¹H-NMR (500 MHz, CDCl₃) δ= -1.47 (s, 3H), -1.44 (s, 1H), 3.81 (s, 3H), 4.01-4.13 (m, 12H), 7.16 (d, *J*= 4.9 Hz, 1H), 7.19 (s, 1H), 7.28 (m, 4H), 7.38 (d, *J*= 8.6 Hz, 2H), 7.46 (m, 2H), 7.56 (d, *J*= 4.9 Hz, 1H), 7.75 (d, *J*= 4.7 Hz, 1H), 7.84 (d, *J*= 8.7 Hz, 2H), 7.91 (d, *J*= 7.0 Hz, 2H), 8.04 (m, 3H), 8.11 (d, *J=* 5.1Hz, 1H), 8.14 (d, *J*= 4.6 Hz, 1H), 8.15 (d, *J*= 5.1 Hz, 1H), 8.40 (d, *J=* 6.4 Hz, 1H); UV-vis (CH₂Cl₂) λₘₐₓ/nm (log ε) 436 (4.78), 486 (4.91), 590 (3.56), 658 (3.74), 824 (4.37); MS (LSIMS) 763 (MH⁺, 100%); HRMS (LSIMS) *m*/*e* calcd for C₅₀H₄₃N₄O₄: 763.32843, found 763.32857 (M⁺); Anal. calcd for C₅₀H₄₂N₄O₄·HIC₅₀H₄₃N₄O₄.I: C, 67.40; H, 4.87; N, 6.29; I, 14.25. Found: C, 67.11; H, 4.97; N, 6.16; I, 14.09.

**N,N'-Dimethylated 2-aza-5,10,15,20-tetra(*m*-methoxyphenyl)-21-carbaporphyrin·HI (compound 12 in Fig. 3).** Yield: 99 mg (82%). R*_{f}*(silica-CH₂Cl₂/5% CH₃OH/2% Et₃N) 0.36; ¹H-NMR (400 MHz, CD₂Cl₂) δ=-1.61 (m, 1H), -1.48 (d, 3×0.7H), -1.39 (s, 3×0.3H), 3.68 (s, 3×0.3H), 3.86 (s, 3×0.7H), 3.95-4.17 (m, 12H), 7.21-8.42 (m, 23H); UV-vis (CH₂Cl₂) λₘₐₓ/nm (log ε) 378 (sh), 478 (4.95), 584 (3.81), 654 (3.74), 724 (sh), 788 (4.38); MS (LSIMS) 763 (MH⁺, 100%); HRMS (LSIMS) *m*/*e* calcd for C₅₀H₄₃N₄O₄: 763.32843, found 763.32854 (MH⁺); Anal. calcd for C₅₀H₄₂N₄O₄·HI·0.5H₂O: C, 66.74; H, 4.93; N, 6.23; I, 14.10. Found: C, 66.72; H, 4.93; N, 6.30; I, 13.97.

### Example 5

### NMR spectroscopic analysis

The structure of **11-III** (see Fig. 6), the major isomer of N,N'-dimethylated 2-aza-5,10,15,20-tetra(*p*-methoxyphenyl)-21-carbaporpliyrin·HI (compound **11** in Fig. 3), was determined by NMR spectroscopic analyses (¹H, ¹³C, selective NOE, HMQC and HMBC). See Fig. 6 for NMR data. The H 3.81 ppm peak was assigned to the H-25 based on the observed cross peak with the C 39.5 ppm peak using HMQC. Selective NOE experiments showed correlations of the H-25 (3.81 ppm) with the H-43a (8.40 ppm) and the H-43b (8.04 ppm), correlation of the H-43a with the H-43b and the H-18 (7.56 ppm) and correlation of the H-18 with the H-17 (7.16 ppm). The 2-N-methyl group hinders rotation of the adjacent *p*-methoxyphenyl group. The rotation is slow and the H-43a and the H-43b can be distinguished by NMR spectroscopy. However, there are still some rotations, which result in the H-43a and the H-43b being interchangeable and their peaks broadened. The negative peak at 8.04 ppm, upon irradiation at 8.40 ppm, showed that the two hydrogens are interchangeable. The -1.47 ppm peak was assigned to the inner methyl group, as suggested by the chemical shift, and integration. The observed cross peaks of C 150.75 ppm with the H-17 (7.16 ppm), the H-18 (7.56 ppm) and the H-26 (-1.47 ppm) in an HMBC experiment clearly showed that the inner methyl group is connected to the pyrrole unite with the H-17 and the H-18.

The structures of the isomers of the other compounds illustrated in Figure 3 may also be determined in a similar way by the above spectroscopic analysis.

### Example 6

### Exemplary embodiments of the invention

Exemplary embodiments of the invention include, but are not limited to, any one or more N,N'-dimethylated N-confused porphyrin compound represented by formulas IV, IV', IV'', IV''', V, V', V', V''', VI, VI', VI'' or VI''' as disclosed above. The labeled and salt forms of said compounds are also exemplary embodiments of the invention, which preferably have groups S¹ through S⁴ as well as groups R³, R⁷, R⁸, R¹², R¹³, R¹⁷ and R¹⁸ defined as disclosed above.

Other exemplary embodiments include a pharmaceutical composition comprising the porphyrin compounds as disclosed above as well as a method of conducting photodynamic therapy in a subject in need thereof comprising administering a porphyrin compound as disclosed herein to said subject and irradiating said subject with at least one wavelength of light suitable to activate said porphyrin. Preferred subjects to be treated by the methods of the invention are human, although animal subjects, whether for agricultural use or human companionship, may also be treated.

### References

1. Furuta, H.; Asano, T.; Ogawa, T. *J. Am. Chem. Soc.* **1994,** 116, 767-768.
2. Chmielewski, P. J.; Latos-Grazynski, L.; Rachlewicz, K.; Glowiac, T. *Angew. Chem., Int. Ed. Engl.* **1994,** 33, 779-781.
3. Chmielewski, P. J.; Latos-Grazynski, L. *J. Chem. Soc., Perk. Trans. 2,* **1995** (3) 503-509.
4. Chmielewski, P. J.; Latos-Grazynski, L.; Glowiak, T. *J. Am. Chem. Soc.* **1996**, 118, 5690-5701.
5. Chmielewski, P. J.; Latos-Grazynski, L.; Schmidt, 1. *Inorg. Chem.* **2000**, 39, 5475-5482.
6. Chmielewski, P. J. and Latos-Grazynski, L. *Inorg. Chem.* **2000**, 39, 5639-5647.
7. Geier, G. R.; Haynes, D. M; Lindsey, J. S. *Org. Lett.* **1999**, 1, 1455-1458.
8. Spiller, W.; Kliesch, H.; Wohrle, D.; Hackbarth, S.; Roder, B.; Schnurpfeil, G. *J. Porphyr. Phthalocya.* **1998**, 2, 145-158.

## Claims

1. An N,N'-dimethylated N-confused porphyrin represented by any one of formulas IV, IV', V, V', VI or VI' as shown below 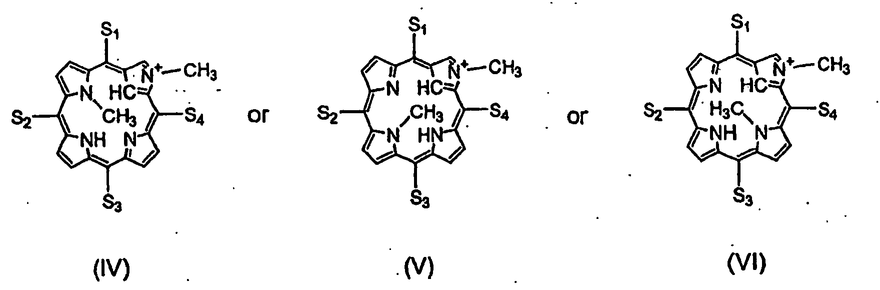 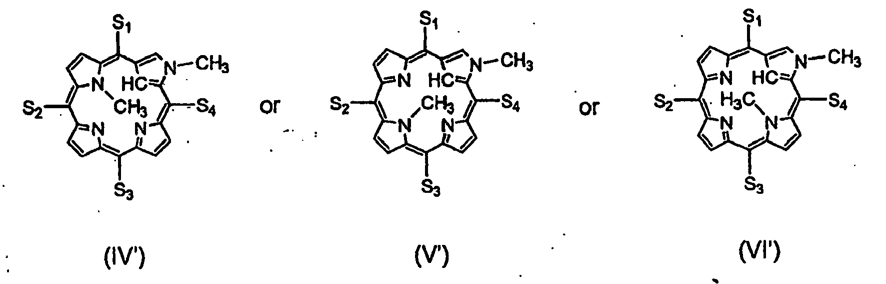 and the labeled and salt forms thereof
wherein S₁ through S₄ are individually selected from H or a group comprising a substituted or unsubstituted alkyl group, cycloalkyl group, aryl ring, aromatic ring, or heterocyclic ring,
wherein, when one or more of S₁ through S₄ is a substituted group, the substitution is selected from a halogen atom; thiol; a carbonyl group; a primary, secondary, tertiary or quaternary amino group; nitrile; a phosphate group and a sulfonate group.

2. A N,N'-dimethylated N-confused porphyrin represented by any one of formulas IV'', IV''', V'', V''', VI'' or VI''' as shown below 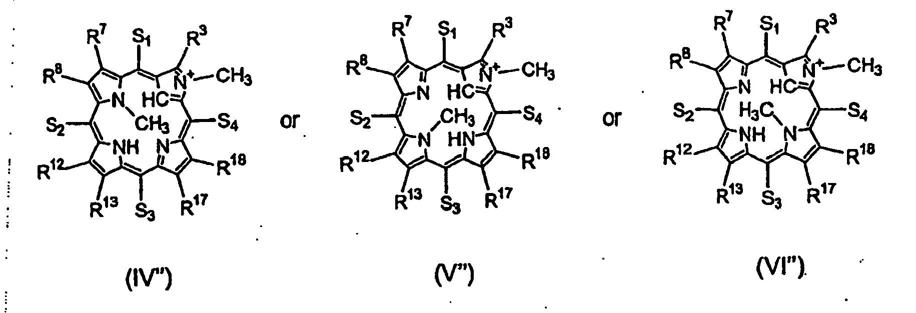 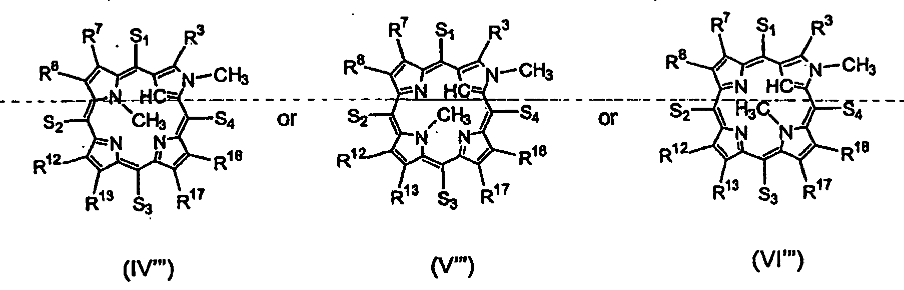 wherein said compound is labeled or in a salt form;
S¹ through S⁴ are individually selected from H or a group comprising a substituted or unsubstituted alkyl group, cycloalkyl group, aryl ring, aromatic ring or heterocyclic ring; are represented by the structure: 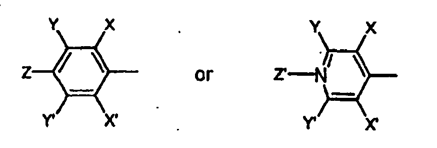 wherein X, Y, Z, X', Y' and Z' are independently selected from hydrogen, halogen, C₁₋₅ alkyl, C₁₋₅ alkoxy, hydroxy, carboxylic acid or acid salt, carboxylic acid ester, sulfonic acid or acid salt, sulfonic acid ester, phosphoric acid, phosphato or phosphate ester, amino, cyano, and nitro wherein, when one or more or S₁ through S₄ is a substituted group, the substitution is selected from a halogen atom; thiol; a carbonyl group; a primary, secondary, tertiary or quaternary amino group; nitrile; a phosphate group and a sulfonate group; and
R³, R⁷, R⁸, R¹², R¹³, R¹⁷ and R¹⁸ are individually selected from H or a group comprising a substituted or unsubstituted alkyl group, alkene containing group, or alkyne containing group; wherein the alkyl, alkene or alkyne containing groups may be optionally and individually substituted by group selected from a halogen atom; thiol; a carbonyl group; a primary, secondary, tertiary or quaternary amino group; nitrile; a phosphate group; and a sulfonate group.

3. The porphyrin according to claim 2 wherein S¹ through S⁴ are individually selected from H;
a C₁₋₁₈ alkyl optionally substituted with a halogen atom; thiol; a carbonyl group; carboxylic acid, ester or amide; a primary, secondary, tertiary, or quaternary amino group; nitrile; a phosphate group; or a sulfonate group;
a C₃₋₇ cycloalkyl, optionally substituted with a halogen atom; thiol; a carbonyl group; carboxylic acid, ester or amide; a primary, secondary, tertiary, or quaternary amino group; nitrile; a phosphate group; or a sulfonate group;
an aromatic ring; or a heterocyclic ring.

4. The porphyrin according to claim 2 wherein S¹ through S⁴ are represented by the structure: 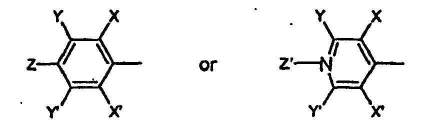 wherein X, Y, Z, X', Y' and Z' are independently selected from hydrogen, halogen, C₁₋₅ alkyl, C₁₋₅ alkoxy, hydroxy, carboxylic acid or acid salt, carboxylic acid ester, sulfonic acid or acid salt, sulfonic acid ester, phosphoric acid, phosphato or phosphate ester, amino, cyano, and nitro.

5. The porphyrin according to any one of claims 2-4 wherein R³, R⁷, R⁸, R¹², R¹³, R¹⁷ and R¹⁸ are individually selected from H or an alkyl, alkene, or alkyne containing group with from 1 to 18 carbon atoms, optionally substituted with a halogen atom; thiol; a carbonyl group; a carboxylic acid or ester or amide; a primary, secondary, tertiary, or quaternary amino group; nitrile; a phosphate group; or a sulfonate group.

6. The porphyrin according to claim 5 wherein said alkyl, alkene, or alkyne containing group has from 1 to 12 carbon atoms, optionally substituted with a halogen atom; thiol; a carbonyl group; a carboxylic acid or ester or amide; a primary, secondary, tertiary, or quaternary amino group; nitrile; a phosphate group; or a sulfonate group.

7. The porphyrin according to claim 6 wherein said alkyl, alkene, or alkyne containing group has from 1 to 6 carbon atoms, optionally substituted with a halogen atom; thiol; a carbonyl group; a carboxylic acid or ester or amide; a primary, secondary, tertiary, or quaternary amino group; nitrile; a phosphate group; or a sulfonate group.

8. The porphyrin according to any one of claims 1-7 as a salt form with CF₃SO₃⁻, I⁻, F⁻, Cl⁻, Br⁻, NO₃⁻, BF₄⁻, CH₃SO₃⁻, CH₃COO⁻ or CF₃COO⁻.

9. A pharmaceutical composition comprising the porphyrin of any one of claims 1-7 and a pharmaceutically acceptable excipient.

10. Use of a porphyrin according to any one of claims 1-7 in the preparation of a medicament for photodynamic therapy of a subject in need thereof.

11. The use of claim 10 wherein said photodynamic therapy reduces unwanted neovasculature in said subject.

12. The use of claim 11 wherein said unwanted neovasculature is in the ocular tissues of said subject.

13. Use of a porphyrin according to any one of claims 1-7 in the preparation of a medicament for photodynamic therapy imaging of tissues and cells of a subject.

14. The use of claim 12 wherein said unwanted neovasculature is in the ocular tissues of said subject.

15. Use of a porphyrin according to any one of claims 1-8 in the preparation of a medicament for photodynamic therapy imaging of tissues and cells of a subject.

## Patentansprüche

1. N,N'-Dimethyliertes, N-invertiertes Porphyrin, dargestellt durch eine der Formeln IV, IV', V, V', VI oder VI', wie nachfolgend gezeigt: 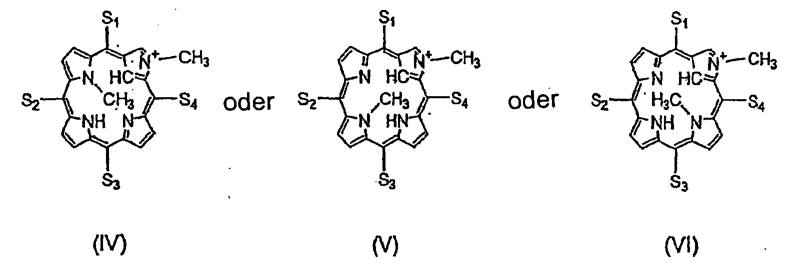 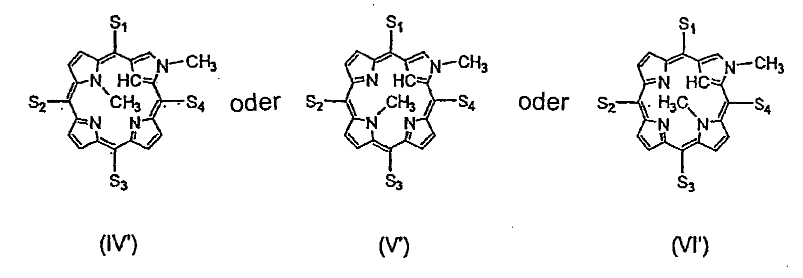 und die markierten und Salzformen davon,
wobei S₁ bis S₄ einzeln aus H oder einer Gruppe, umfassend eine(n) substituierte(n) oder unsubstituierte(n) Alkylgruppe, Cycloalkylgruppe, Arylring, aromatischen Ring oder heterocyclischen Ring, ausgewählt sind, wobei, wenn eines oder mehrere von S₁ bis S₄ eine substituierte Gruppe ist, die Substitution aus einem Halogenatom, Thiol, einer Carbonylgruppe, einer primären, sekundären, tertiären oder quartären Aminogruppe, Nitril, einer Phosphatgruppe und einer Sulfonatgruppe ausgewählt ist.

2. N,N'-Dimethyliertes, N-invertiertes Porphyrin, dargestellt durch eine der Formeln IV'', IV''', V", V"', VI'' oder VI''', wie nachfolgend gezeigt: 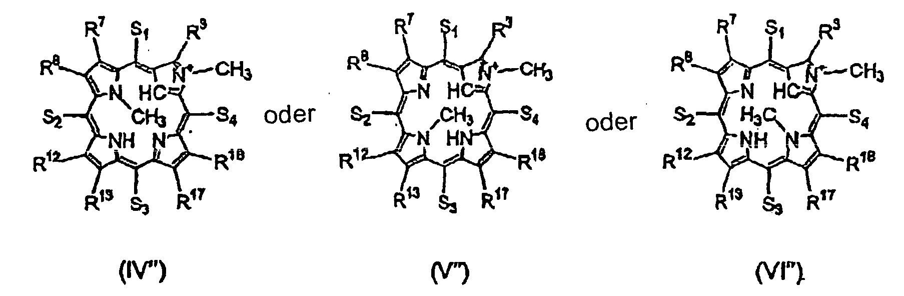 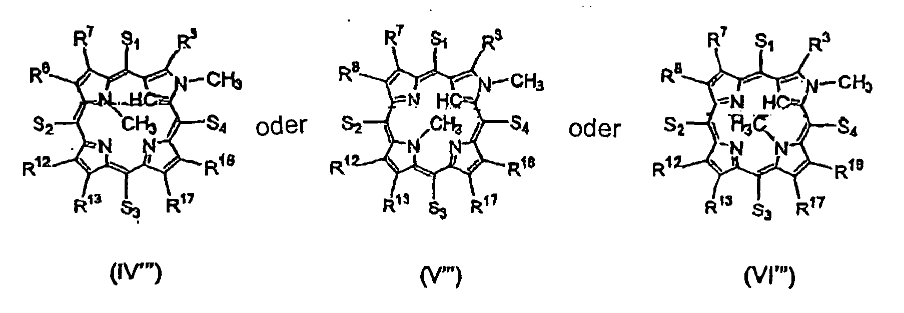 wobei die Verbindung markiert oder in einer Salzform vorliegt,
S¹ bis S⁴ einzeln aus H oder einer Gruppe, umfassend eine(n) substituierte(n) oder unsubstituierte(n) Alkylgruppe, Cycloalkylgruppe, Arylring, aromatischen Ring oder heterocyclischen Ring, dargestellt durch die Struktur: 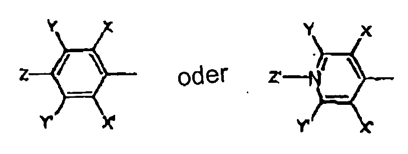 ausgewählt sind, wobei X, Y, Z, X', Y' und Z' unabhängig aus Wasserstoff, Halogen, C₁₋₅-Alkyl, C₁₋₅-Alkoxy, Hydroxy, Carbonsäure oder -säuresalz, Carbonsäureester, Sulfonsäure oder -säuresalz, Sulfonsäureester, Phosphorsäure, Phosphato- oder Phosphatester, Amino, Cyano und Nitro ausgewählt sind, wobei, wenn eines oder mehrere von S₁ bis S₄ eine substituierte Gruppe ist, die Substitution aus einem Halogenatom, Thiol, einer Carbonylgruppe, einer primären, sekundären, tertiären oder quartären Aminogruppe, Nitril, einer Phosphatgruppe und einer Sulfonatgruppe ausgewählt ist, und R³, R⁷, R⁸, R¹², R¹³, R¹⁷ und R¹⁸ einzeln aus H oder einer Gruppe, umfassend eine substituierte oder unsubstituierte Alkylgruppe, Alken-haltige Gruppe oder Alkin-haltige Gruppe, ausgewählt sind, wobei die Alkyl-, Alken- oder Alkin-haltigen Gruppen gegebenenfalls und einzeln mit einer Gruppe, ausgewählt aus einem Halogenatom, Thiol, einer Carbonylgruppe, einer primären, sekundären, tertiären oder quartären Aminogruppe, Nitril, einer Phosphatgruppe und einer Sulfonatgruppe, substituiert sein können.

3. Porphyrin nach Anspruch 2, wobei S¹ bis S⁴ einzeln aus H,
einem C₁₋₁₈-Alkyl, gegebenenfalls substituiert mit einem Halogenatom, Thiol, einer Carbonylgruppe, Carbonsäure, -ester oder -amid, einer primären, sekundären, tertiären oder quartären Aminogruppe, Nitril, einer Phosphatgruppe oder einer Sulfonatgruppe,
einem C₃₋₇-Cycloalkyl, gegebenenfalls substituiert mit einem Halogenatom, Thiol, einer Carbonylgruppe, Carbonsäure, -ester oder -amid, einer primären, sekundären, tertiären oder quartären Aminogruppe, Nitril, einer Phosphatgruppe oder einer Sulfonatgruppe,
einem aromatischen Ring oder einem heterocyclischen Ring ausgewählt sind.

4. Porphyrin nach Anspruch 2, wobei S¹ bis S⁴ durch die Struktur: 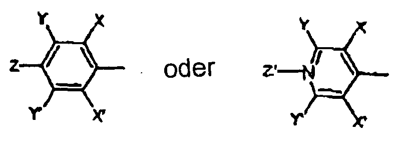 dargestellt sind, wobei X, Y, Z, X', Y' und Z' unabhängig aus Wasserstoff, Halogen, C₁₋₅-Alkyl, C₁₋₅-Alkoxy, Hydroxy, Carbonsäure oder -säuresalz, Carbonsäureester, Sulfonsäure oder -säuresalz, Sulfonsäureester, Phosphorsäure, Phosphato- oder Phosphatester, Amino, Cyano und Nitro ausgewählt sind.

5. Porphyrin nach einem der Ansprüche 2 bis 4, wobei R³, R⁷, R⁸, R¹², R¹³, R¹⁷ und R¹⁸ einzeln aus H oder einer Alkyl-, Alken- oder Alkin-haltigen Gruppe mit von 1 bis 18 Kohlenstoffatomen, gegebenenfalls substituiert mit einem Halogenatom, Thiol, einer Carbonylgruppe, einer bzw. einem Carbonsäure oder -ester oder -amid, einer primären, sekundären, tertiären oder quartären Aminogruppe, Nitril, einer Phosphatgruppe oder einer Sulfonatgruppe, ausgewählt sind.

6. Porphyrin nach Anspruch 5, wobei die Alkyl-, Alken- oder Alkin-haltige Gruppe von 1 bis 12 Kohlenstoffatome aufweist, gegebenenfalls substituiert mit einem Halogenatom, Thiol, einer Carbonylgruppe, einer bzw. einem Carbonsäure oder -ester oder -amid, einer primären, sekundären, tertiären oder quartären Aminogruppe, Nitril, einer Phosphatgruppe oder einer Sulfonatgruppe.

7. Porphyrin nach Anspruch 6, wobei die Alkyl-, Alken- oder Alkin-haltige Gruppe von 1 bis 6 Kohlenstoffatome aufweist, gegebenenfalls substituiert mit einem Halogenatom, Thiol, einer Carbonylgruppe, einer bzw. einem Carbonsäure oder -ester oder -amid, einer primären, sekundären, tertiären oder quartären Aminogruppe, Nitril, einer Phosphatgruppe oder einer Sulfonatgruppe.

8. Porphyrin nach einem der Ansprüche 1 bis 7 als eine Salzform mit CF₃SO₃⁻, I⁻, F⁻, Cl⁻, Br⁻, NO₃⁻, BF₄⁻, CH₃SO₃⁻, CH₃COO⁻ oder CF₃COO⁻.

9. Pharmazeutische Zusammensetzung, umfassend das Porphyrin nach einem der Ansprüche 1 bis 7 und ein pharmazeutisch verträglicher Arzneimittelträger.

10. Verwendung eines Porphyrins nach einem der Ansprüche 1 bis 7 in der Herstellung eines Medikaments zur photodynamischen Therapie eines dessen bedürftigen Patienten.

11. Verwendung nach Anspruch 10, wobei die photodynamische Therapie unerwünschte Neovaskulatur in dem Patienten verringert.

12. Verwendung nach Anspruch 11, wobei die unerwünschte Neovaskulatur in den Okulargeweben des Patienten vorliegt.

13. Verwendung eines Porphyrins nach einem der Ansprüche 1 bis 7 in der Herstellung eines Medikaments zur photodynamischen Therapie-Abbildung von Geweben und Zellen eines Patienten.

14. Verwendung nach Anspruch 12, wobei die unerwünschte Neovaskulatur in den Okulargeweben des Patienten vorliegt.

15. Verwendung eines Porphyrins nach einem der Ansprüche 1 bis 8 in der Herstellung eines Medikaments zur photodynamischen Therapie-Abbildung von Geweben und Zellen eines Patienten.

## Revendications

1. Porphyrine N,N'-diméthylée, à enchevêtrement N, représentée par l'une quelconque des formules IV, IV', V, V', VI et VI' telles que représentées ci-dessous : 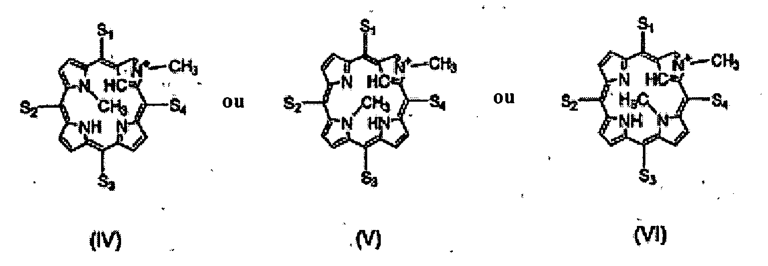 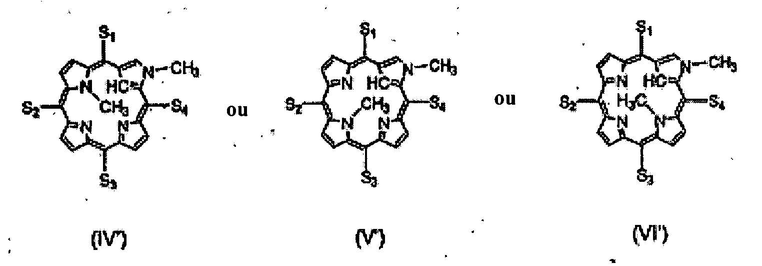 et les formes marquées et les formes sels de celle-ci,
dans laquelle les radicaux S₁ à S₄ sont choisis individuellement entre H et le groupe constitué par un groupe alkyle substitué ou non substitué, un groupe cycloalkyle, un cycle aryle, un cycle aromatique ou un cycle hétérocyclique dans lequel, lorsque un ou plusieurs des radicaux S₁ à S₄ représente(nt) un groupe substitué, la substitution est choisie entre un atome d'halogène ; un groupe thiol ; un groupe carbonyle ; un groupe amino primaire, secondaire, tertiaire ou quaternaire ; un groupe nitrile ; un groupe phosphate et un groupe sulfonate.

2. Porphyrine N-N'-diméthylée, à enchevêtrement N, représentée par l'une quelconque des formules IV", IV"', V", V''', VI" et VI''' telles que représentées ci-dessous : 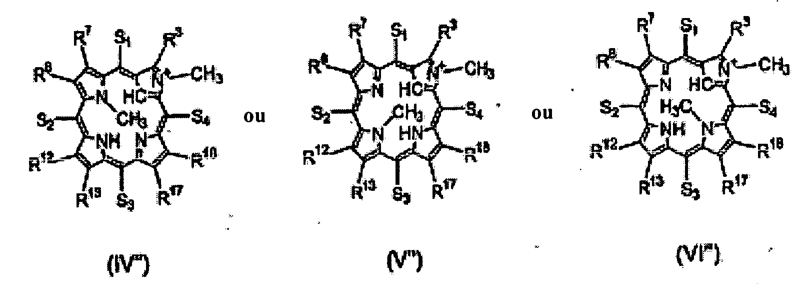 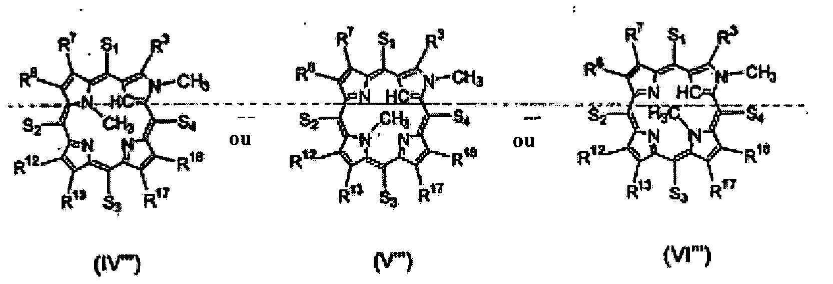 dans laquelle ledit composé est sous forme marquée ou sous forme de sel ;
les radicaux S₁ à S₄ sont choisis individuellement entre H et le groupe constitué par un groupe alkyle substitué ou non substitué, un groupe cycloalkyle, un cycle aryle, un cycle aromatique ou un cycle hétérocyclique ; et représentés par la structure : 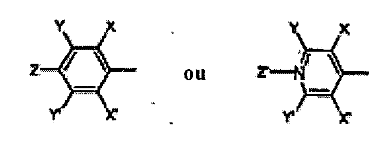 dans laquelle X, Y, Z X', Y' et Z' sont choisis indépendamment entre l'hydrogène, un halogène, un groupe alkyle en C₁ à C₅, alkoxy en C₁ à C₅, hydroxy, un acide ou un sel d'acide carboxylique, un ester d'acide carboxylique, un acide ou un sel d'acide sulfonique, un ester d'acide sulfonique, un acide phosphorique, un phosphate ou un ester de phosphate, un groupe amino, cyano et nitro, dans lesquels lorsqu'un ou plusieurs radicaux S₁ à S₄ représente(nt) un groupe substitué, la substitution est choisie entre un atome d'halogène ; un groupe thiol ; un groupe carbonyle ; un groupe amino primaire, secondaire, tertiaire ou quaternaire ; un groupe nitrile ; un groupe phosphate et un groupe sulfonate ; et
R³, R⁷, R⁸, R¹², R¹³, R¹⁷ et R¹⁸ sont choisis individuellement entre H et le groupe constitué par un groupe alkyle substitué ou non substitué, un groupe contenant un groupe alcène ou un groupe contenant un groupe alcyne, les groupes contenant des groupes alkyle, alcène ou alcyne pouvant être facultativement et individuellement substitués par un groupe choisi parmi un atome d'halogène ; un groupe thiol ; un groupe carbonyle ; un groupe amino primaire, secondaire, tertiaire ou quaternaire ; un groupe nitrile ; un groupe phosphate ; et un groupe sulfonate.

3. Porphyrine selon la revendication 2, dans laquelle les radicaux S¹ à S⁴ sont choisis individuellement entre H ;
un groupe alkyle en C₁ à C₁₈ facultativement substitué par un atome d'halogène ; un groupe thiol ; un groupe carbonyle ; un acide, ester ou amide carboxylique ; un groupe amino primaire, secondaire, tertiaire ou quaternaire ; un groupe nitrile ; un groupe phosphate ; ou un groupe sulfonate ;
un groupe cycloalkyle en C₃ à C₇ facultativement substitué par un atome d'halogène ; un groupe thiol ; un groupe carbonyle ; un acide, ester ou amide carboxylique ; un groupe amino primaire, secondaire, tertiaire ou quaternaire ; un groupe nitrile ; un groupe phosphate ou un groupe sulfonate ;
un cycle aromatique ; ou un cycle hétérocyclique.

4. Porphyrine selon la revendication 2, dans laquelle les radicaux S¹ à S⁴ sont représentés par la structure : 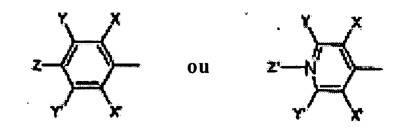 dans laquelle X, Y, Z, X', Y' et Z' sont choisis indépendamment entre l'hydrogène, un halogène, un groupe alkyle en C₁ à C₅, alkoxy en C₁ à C₅, hydroxy, un acide ou un sel d'acide carboxylique, un ester d'acide carboxylique, un acide ou un sel d'acide sulfonique, un ester d'acide sulfonique, un acide phosphorique, un groupe phosphate ou un ester de phosphate, un groupe amino, cyano et nitro.

5. Porphyrine selon l'une quelconque des revendications 2 - 4, dans laquelle R³, R⁷, R⁸, R¹², R¹³, R¹⁷ et R¹⁸ sont choisis individuellement entre H et un groupe contenant un groupe alkyle, alcène ou alcyne ayant 1 à 18 atomes de carbone, facultativement substitué par un atome d'halogène ; un groupe thiol ; un groupe carbonyle ; un acide, ester ou amide d'acide carboxylique ; un groupe amino primaire, secondaire, tertiaire ou quaternaire ; un groupe nitrile ; un groupe phosphate ; ou un groupe sulfonate.

6. Porphyrine selon la revendication 5, dans laquelle ledit groupe contenant un groupe alkyle, alcène ou alcyne présente 1 à 12 atomes de carbone, facultativement substitués par un atome d'halogène ; un groupe thiol ; un groupe carbonyle ; un acide, ester ou amide d'acide carboxylique ; un groupe amino primaire, secondaire, tertiaire ou quaternaire ; un groupe nitrile ; un groupe phosphate ; ou un groupe sulfonate.

7. Porphyrine selon la revendication 6, dans laquelle ledit groupe contenant un groupe alkyle, alcène ou alcyne présente 1 à 6 atomes de carbone, facultativement substitués par un atome d'halogène ; un groupe thiol ; un groupe carbonyle ; un acide, ester ou amide d'acide carboxylique ; un groupe amino primaire, secondaire, tertiaire ou quaternaire ; un groupe nitrile ; un groupe phosphate ; ou un groupe sulfonate.

8. Porphyrine selon l'une quelconque des revendications 1 à 7, sous forme de sel avec CF₃SO₃⁻, I⁻, F⁻, Cl⁻, Br⁻, NO₃⁻, BF₄⁻, CH₃SO₃⁻, CH₃COO⁻ou CF₃COO⁻.

9. Composition pharmaceutique comprenant la porphyrine selon l'une quelconque des revendications 1 - 7 et un excipient pharmaceutiquement acceptable.

10. Utilisation d'une porphyrine selon l'une quelconque des revendications 1 - 7 dans la préparation d'un médicament pour la thérapie photodynamique d'un patient nécessitant un tel traitement.

11. Utilisation selon la revendication 10, dans laquelle ladite thérapie photodynamique réduit une néovascularisation indésirable dudit patient.

12. Utilisation selon la revendication 11, dans laquelle ladite néovascularisation indésirable se situe dans les tissus oculaires dudit patient.

13. Utilisation d'une porphyrine selon l'une quelconque des revendications 1 - 7 dans la préparation d'un médicament pour l'imagerie d'une thérapie photodynamique de tissus et de cellules d'un patient.

14. Utilisation selon la revendication 12, dans laquelle ladite néovascularisation indésirable se situe dans les tissus oculaires dudit patient.

15. Utilisation d'une porphyrine selon l'une quelconque des revendications 1 - 8 dans la préparation d'un médicament pour l'imagerie d'une thérapie photodynamique de tissus et de cellules d'un patient.
